(19) Europäisches Patentamt
European Patent Office
Office européen des brevets

(11) **EP 4 732 766 A1**

(12) **EUROPEAN PATENT APPLICATION**

(43) Date of publication:
**29.04.2026 Bulletin 2026/18**

(21) Application number: **25211687.6**

(22) Date of filing: **28.10.2025**

(51) International Patent Classification (IPC):
**A61B 5/053** (2021.01)    **A61B 5/145** (2006.01)
**A61B 5/01** (2006.01)

(52) Cooperative Patent Classification (CPC):
**A61B 5/14517; A61B 5/0533; A61B 5/0537;
A61B 5/4266;** A61B 5/01; A61B 5/486

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **28.10.2024 US 202463712660 P
21.05.2025 US 202519214759**

(71) Applicant: **Epicore Biosystems, Inc.
Cambridge, MA 02139 (US)**

(72) Inventors:
• **WRIGHT, Donald E.
Boston, 02127 (US)**
• **SULESKI, Brandon J.
Cambridge, 02140 (US)**
• **LEE, Stephen P.
Ann Arbor, 481004 (US)**
• **LI, Weihua
Acton, 01720 (US)**

• **SPINELLI, Julia C.
Cambridge, 02140 (US)**
• **DIZOGLIO, Thomas
Brattleboro, 05301 (US)**
• **SCARTH, Alan P.
Winnipeg, R3M-0P5 (US)**
• **ARANYOSI, Alexander J.
Medford, 02143 (US)**
• **GHAFFARI, Roozbeh
Cambridge, 02142 (US)**
• **YANG, Kaitao
West Newton, 01465 (US)**
• **MACINTOSH, David E.
Orlando, 32803 (US)**
• **WRIGHT JR., John A.
Lexington, 02421 (US)**

(74) Representative: **Haseltine Lake Kempner LLP
Cheapside House
138 Cheapside
London EC2V 6BJ (GB)**

(54) **BIOFLUID COMPUTATION MEASUREMENT SYSTEM WITH RE-USABLE SKIN FACING ELECTRODES**

(57)    An electrode substrate is configured to be pressed onto the skin of a wearer and includes a body having skin-facing side and a second side that faces away from the skin. A plurality of raised platforms extend outwardly from the skin-facing side, and recessed areas are formed between each of the raised platforms and define air gaps for the flow of air between each raised platform, and wherein edges of the raised platforms are rounded for comfort when worn and pressed into the skin of the wearer. A skin-facing electrode is formed on each of at least two of the raised platforms. The skin-facing electrodes are planar and flush with an outer surface of the raised platforms. The electrode substrate and the skin-facing electrodes formed thereon are configured to measure change in impedance of the skin onto which it is pressed over time before, during, and after sweat perfusion.

FIG. 1

**Description**

CROSS-REFERENCE TO RELATED APPLICATIONS

This application claims the benefit of United States Provisional Application No. 63/712,660, filed October 28, 2024, the disclosure of which is incorporated herein by reference.

BACKGROUND OF THE INVENTION

This invention relates in general to wearable fluidic systems for collecting, measuring, and/or monitoring sweat rate, sweat loss, sweat volume, sweat composition, and/or biochemical information about one or more persons. In particular, this invention relates to an improved, wearable, and re-usable biofluid computation measurement system that measures electro-dermal activity (EDA), sweat metrics, and physiological parameters to generate a unique, characteristic, sweat profile for an individual and then use the sweat profile generated to enhance the measured EDA.

Dehydration and exposure to excessive heat during physical activity creates occupational safety risks, giving rise to deficits in physical and cognitive functions, and reduced work capacity. Proper hydration is the single most important and economically feasible mitigation strategy against occupational heat strain. Real-time monitoring of sweat and biophysical markers can offer valuable physiological insights about heat stress and related thermoregulatory response, particularly for individuals exposed to heat. These metrics, however, have proven to be challenging to achieve with wearable sensors.

Most existing sweat sensors are designed for short exercise periods, such as durations of less than 4 hours, and specifically measure the initial rate of sweat. These sweat sensors are known to fail to measure for longer periods, such as measuring sweat loss during all or most of a work day, or for endurance athletes. Many known sweat sensors require adhesive to adhere to the skin to fill a fluidic patch, and are not always convenient. Such sensors are further affected by a phenomenon called hidromeiosis, wherein residual sweat pooled near the device inlet suppresses the local sweating response for subsequent bouts of sweating.

One approach to measuring sweat production is to apply a plurality of electrodes to the skin to measure electro-dermal activity (EDA), a complex electrical signal produced by a combination of neural activity, sweat gland activity, changes of skin conductance due to the presence of electrolyte-containing sweat, and other sources of electrical signals. Some existing methods of sweat measurement may use EDA electrodes to measure skin moisture, but these methods are not designed to measure changing sweat volumes over time, nor are they designed to measure electrolyte levels.

Thus, it would be desirable to provide an improved, wearable, and reusable biofluid computation measurement system that measures EDA, sweat metrics, and physiological parameters to generate a unique, characteristic, sweat profile for an individual and then to use the sweat profile generated to enhance the measured EDA.

SUMMARY OF THE INVENTION

This invention relates to an improved wearable and re-usable biofluid computation measurement system that measures electro-dermal activity (EDA), sweat metrics, and physiological parameters to generate a unique, characteristic, sweat profile for an individual and then use the sweat profile generated to enhance the measured EDA. In one embodiment, an electrode substrate is configured to be pressed onto the skin of a wearer and includes a body having skin-facing side and a second side that faces away from the skin. A plurality of raised platforms extend outwardly from the skin-facing side, and recessed areas are formed between each of the raised platforms and define air gaps for the flow of air between each raised platform, and wherein edges of the raised platforms are rounded for comfort when worn and pressed into the skin of the wearer. A skin-facing electrode is formed on each of at least two of the raised platforms. The skin-facing electrodes are planar and flush with an outer surface of the raised platforms. The electrode substrate and the skin-facing electrodes formed thereon are configured to measure change in impedance of the skin onto which it is pressed over time before, during, and after sweat perfusion.

In another embodiment, a sweat rate, volume, and electrolyte measurement system includes a wearable biofluid computation measurement device including: a re-usable electro-dermal activity (EDA) band assembly having an electrode substrate mounted within a substrate housing portion of a clip, and a compression strap attached to the clip; and a re-usable electronic module attached to the EDA band assembly. The sweat rate, volume, and electrolyte measurement system further includes a smart device. The electrode substrate has at least one pair of a skin-facing electrodes formed thereon. The electronic module includes one of a haptic alert and an alarm that activates when the sensor readings from the electrode substrate exceed a pre-determined threshold measurements including, but not limited to, sweat volume, sweat deficit, defined as sweat volume lost minus fluid intake, and core-temperature. The measurement taken by the electrode substrate are wirelessly transmitted to the smart device.

Various aspects of this invention will become apparent to those skilled in the art from the following detailed description of the preferred embodiment, when read in view of the accompanying drawings.

## BRIEF DESCRIPTION OF THE DRAWINGS

Fig. 1 is a top plan view of a first embodiment of a biofluid computation measurement system showing the reusable electronic module mounted to the EDA band assembly and further showing the biofluid computation measurement system worn on a human arm.

Fig. 2 is a top plan view of the electrode substrate mounted within the clip shown in Fig. 1, with the strap removed.

Fig. 3A is a bottom plan view of the electrode substrate mounted within the clip illustrated in Fig. 2.

Fig. 3B is an enlarged bottom plan view of a first embodiment of the electrode substrate illustrated in Fig. 3A, with the clip removed.

Fig. 3C is an enlarged top plan view of the electrode substrate illustrated in Figs. 3A and 3B, with the clip removed.

Fig. 3D is a side elevational view of the electrode substrate illustrated in Figs. 3A through 3C.

Fig. 4A is a top plan view of the reusable electronic module shown in Fig. 1.

Fig. 4B is a bottom plan view of the reusable electronic module shown in Figs. 1 and 4A.

Fig. 5A is a top plan view of a one-time use fluidic patch.

Fig. 5B is bottom plan view of the one-time use fluidic patch illustrated in Fig. 5A.

Fig. 6 is an exploded perspective view of the reusable electronic module shown in Figs. 1, 4A, and 4B.

Fig. 7 is a bottom plan view of a second embodiment of the electrode substrate mounted within the clip illustrated in Figs. 2 and 3A.

Fig. 8 is a schematic diagram of a two-point measurement circuit using a transimpedance amplifier topology.

Fig. 9 is a schematic diagram illustrating how the resistance between the electrodes corresponds to the voltage of the transimpedance amplifier.

Fig. 10 is a schematic diagram of a four-point measurement circuit topology.

Fig. 11 is a graph of an electrical conductance signal from a pair of skin-engaging electrodes (mV) vs. time (minutes).

Fig. 12 is a schematic cross-sectional view of human epidermis and dermis showing skin-facing electrodes in contact with the epidermis and current paths vs. skin depth for varying levels of excitation.

Fig. 13 is a graph of an electrical conductance signal from pairs of skin-engaging electrodes (mV) at different electrode pitches vs. time (seconds).

Fig. 14 is a perspective view of a first alternate embodiment of the clip shown in Figs. 1, 2, and 3A showing castellations defining airflow passages.

Fig. 15 is a perspective view of a second alternate embodiment of the clip shown in Figs. 1, 2, and 3A showing airflow slots in a side wall of the substrate housing portion of the clip.

Fig. 16A is a graph comparing sodium cavity plateau voltage vs. measured sodium concentration.

Figs. 16B through 16E are graphs of electrical conductance voltage of captured sweat in a cavity of known volume vs. time for four unique subjects with different sweat sodium concentration levels.

Fig. 17 is a graph of an electrical conductance signal of artificial skin being perfused at constant rate with sodium saline solutions of varying concentrations.

Fig. 18A is a flow chart illustrating the process of using only the disposable fluidic patch with the module and a smartphone.

Fig. 18B is a flow chart illustrating the process of first using the disposable fluidic patch with the module and a smartphone to build a characteristic sweat profile, then, after a training period, switching to the re-usable EDA band assembly.

Fig. 18C is a flow chart illustrating the process of using only the EDA band assembly with the module and a smartphone.

Fig. 19 is a data flow chart of the module initialization with focus on the EDA mode, identifying which accessory is attached, determining if a user sweat profile exists, and loading the profile to perform an enhanced measurement.

Fig. 20A is a plan view of a first portion of a GUI App screen.

Fig. 20B is a plan view of a second portion of the GUI App screen shown in Fig. 20A.

Fig. 20C is a plan view of a third portion of the GUI App screen shown in Figs. 20A and 20B.

Fig. 21 is a plan view of a GUI App screen showing a method to encourage users to build a sweat profile.

Fig. 22 is an enlarged bottom plan view of a third embodiment of the electrode substrate, showing multiple wells of increasing volumes.

Fig. 23 is a cross-sectional view taken along the line 23 - 23 of Fig. 22, showing one of the wells illustrated in Fig. 22.

Fig. 24A is a bottom plan view of a fourth embodiment of the electrode substrate, showing two wells, each within a dedicated raised platform.

Fig. 24B is a top plan view of the electrode substrate illustrated in Fig. 24A.

Fig. 24C is a cross-sectional view taken along the line 24C - 24C of Fig. 24A.

Fig. 24D is an enlarged cross-sectional view taken from circle D in Fig. 23C.

Fig. 25A is plan view of a first portion of the user screen in the cloud web portal.

Fig. 25B is a plan view of a second portion of the user screen in the cloud web portal.

DETAILED DESCRIPTION OF THE PREFERRED EMBODIMENT

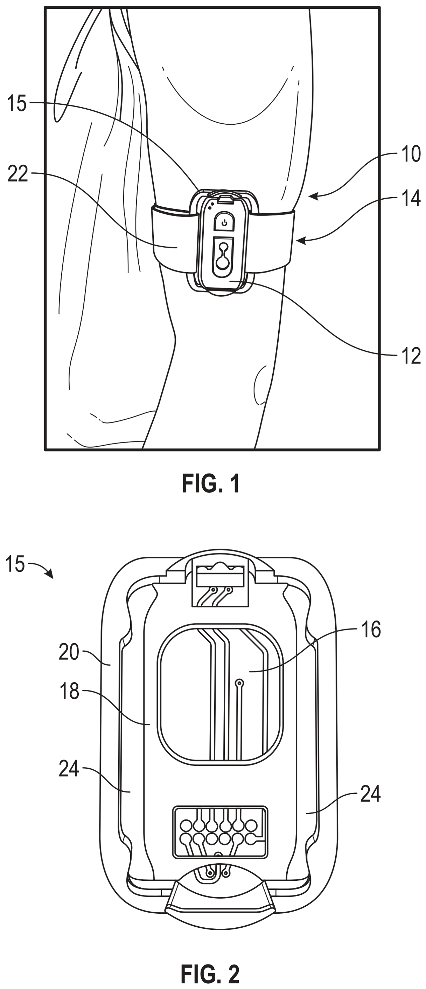

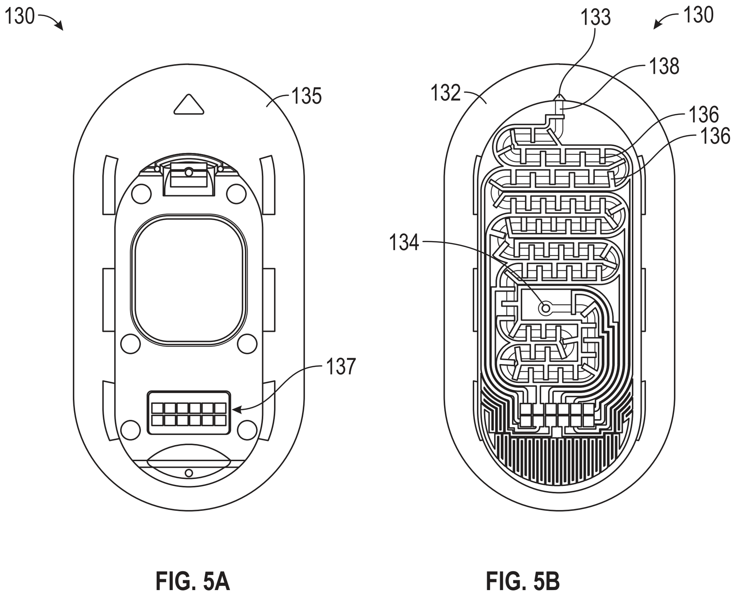

The following definitions are provided to clarify their specific use in the context of the invention.
The terms fluidic substrate, and microfluidic substrate, refer to the substrate component of the biofluid computation measurement device having at least one function or purpose other than providing mechanical support for a component or components disposed on, or within, the substrate exhibiting microfluidic functionality, such as providing transport of a bodily fluid through, or within, the substrate, for example via spontaneous capillary action, or via an active actuation.
The term fluidic channel refers to a groove or passage for fluid, such as sweat, to flow.
The term fluid reservoir refers to a recess or cavity into which fluid, such as sweat, may flow.
The term skin-facing electrode refers to an electrode that makes direct, physical contact with skin. This contrasts with electrodes that do not make direct, physical contact with skin, such as electrodes that are inside a channel or reservoir, and only make direct, physical contact with perfused sweat.
The term skin-facing electrode substrate refers to the substrate component of the biofluid computation measurement device having at least one function or purpose other than providing mechanical support for a component or components disposed on, or within, the substrate exhibiting electrical conductivity functionality, such as electrodes that make contact with skin and can conduct current, measure or apply a voltage, and measure the resulting current.
The term smart device refers to a wirelessly connected device that can have custom applications from third party providers, including, but not limited to, a smartphone, such as an iPhone®, or a phone running the Android™ operating system, a tablet, such as an iPad®, or a smart watch, such as an Apple® watch.
The term EDA refers to electro-dermal activity which is the combined electrical activity that can be measured from the surface of the skin, including measured electrical resistance, impedance, conductance, and voltage potential of skin as it responds to variation in sweat gland activity. Historically, EDA has been referred to as galvanic skin response (GSR) and electro-dermal response (EDR).
The term module refers to the encased electronics that contain a microcontroller, battery, haptic vibration motor, interactive buttons, and sensing circuitry that interfaces with a fluidic patch or an EDA band assembly.
The term fluidic patch refers to an adhesive patch that contains a fluidic or microfluidic substrate that contains a fluidic channel. The fluidic patch adheres to the skin to capture perfused sweat from the skin pores.
The term EDA band assembly refers to an accessory or assembly that compresses a skin-facing electrode substrate onto the skin of a wearer by means of a band, such as an elastic band.
The term session refers to a single block of time in which the module is used. A session typically corresponds to a work day, a work shift, or, in athletics, a practice session or a game.
The term biofluid refers to sweat, but may also refer to biological fluids that can be excreted from the body such as urine, secreted fluids, such as breast milk and/or wound exudate, or obtained with a needle such as interstitial sweat fluid or blood.
Advantageously, the biofluid computation measurement systems described herein below are structured and configured to systematically measure changing sweat rates over time, and make the sweat rate data actionable for a person, such as safety health person, or nutritionist/coach, to take action based on the sweat rate data in an organization.
Referring now to Figs. 1 through 5B, a first embodiment of a biofluid computation measurement device is shown at 10. The biofluid computation measurement device 10 is a wearable, sweat rate, volume, and electrolyte measurement device that includes a reusable electronic module 12 mounted to a re-usable electro-dermal activity (EDA) band assembly 14. The biofluid computation measurement device 10 may be combined with a smart device, such as a smartphone 180 to display the level of physical activity, sweat rate, sweat volume, electrolyte level, skin temperature, and other bodily functions computed by the computation measurement system.

The re-usable EDA band assembly 14 includes an electrode substrate 16 mounted within a substrate housing portion 18 of a clip 20. When the electrode substrate 16 is formed with, or otherwise mounted to, the clip 20, a clip assembly 15 is defined. A compression strap 22 is attached to the clip 20 via slots 24. In the illustrated embodiment, the electrode substrate 16 is integrally formed within the substrate housing portion 18 of the clip 20, such as during a molding process. The electrode substrate 16 may be mounted within the substrate housing portion 18 of the clip 20 by other means, including, but not limited to, with fasteners, such as threaded fasteners, adhesive, or with a snap-fit attachment.

The compression strap 22 may be formed from any desired material, such as a stretchable, breathable material that allows for comfortable yet taut tension on the user that ensures that the electrode substrate 16 maintains contact with skin. If desired, the skin-facing surface of the compression strap 22 may have rubber microdots formed thereon to help retain the biofluid computation measurement device 10 in a desired position on the user's arm, such as shown in Fig. 1. Additionally, the compression strap 22 may be configured such that the amount of tension applied to the compression strap 22 is controlled, thus allowing the pressure of the electrode substrate 16 on the skin to be controlled.

Although the biofluid computation measurement device 10 is shown worn on the user's arm, it will be understood that the biofluid computation measurement device 10 may be configured to be worn at any desired location on the user's body, including, but not limited to, the forearm, the leg, the ankle, the torso, and the head, such as within a hat or other headgear. Additionally, the biofluid computation measurement device 10 may be incorporated into a worn smart device, such as a smart watch or a smart ring.

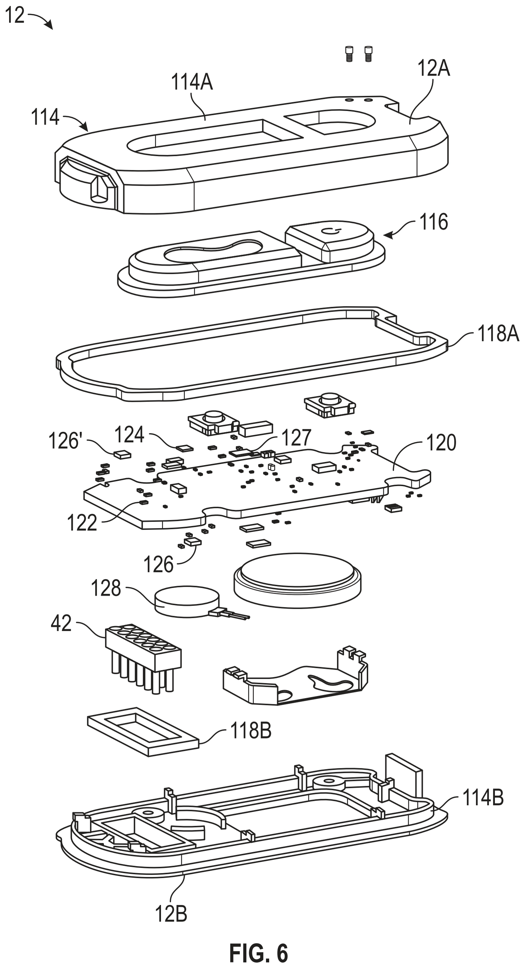

The re-usable electronic module 12, also shown in Fig. 6 and described in detail below, includes a first or outwardly facing side 12A and a second side 12B facing the EDA band assembly 14. The second side includes a plurality of spring-loaded pins defining a spring connector 42. The spring connector is configured to provide an electrical connection to the electrode substrate 16 of the EDA band assembly 14.

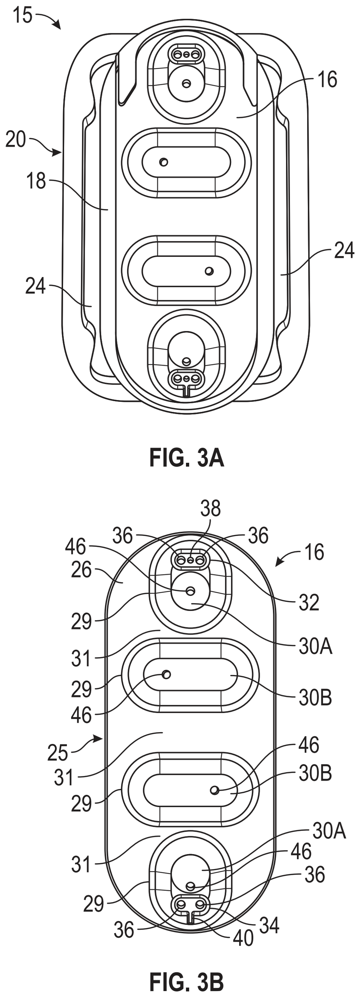

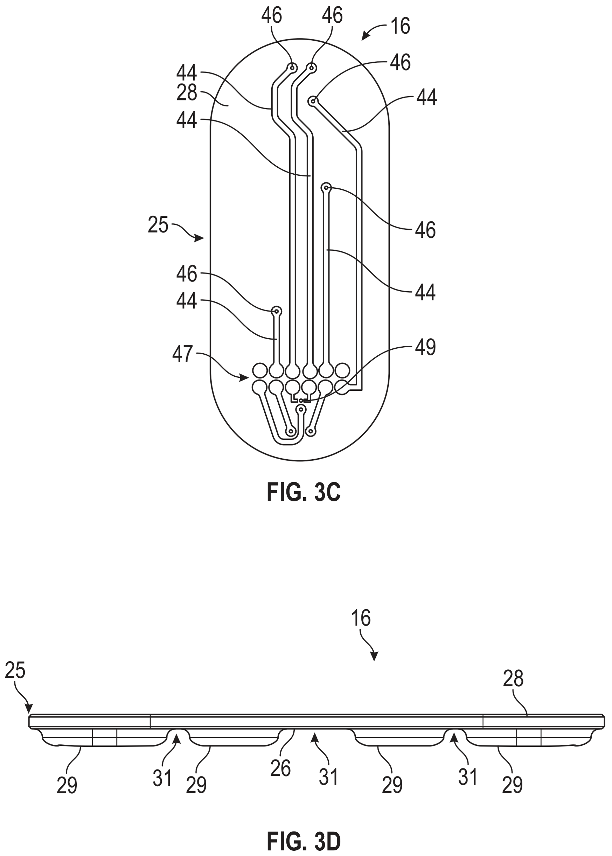

The first embodiment of the electrode substrate 16, best shown in Figs. 3A through 3D includes a body 25 having a first or skin-facing side 26 and a second side 28 that faces away from the skin. The electrode substrate 16 may be formed out of sintered ceramic, such as, but not limited to, alumina. Alternatively, the electrode substrate 16 may be formed out of injection molded polymers. Electrodes and traces may be metallized using techniques such as, but not limited to, electroplating and vapor deposition. Alternatively, a PCB (not shown) may be mounted within the body 24. The skin-facing side 26 includes raised platforms 29 upon which pill-shaped, skin-contacting electrodes 30A and 30B are formed, i.e., the platforms 29 extend outwardly from the body 24 of the electrode substrate 16. The skin-facing side 26 further includes a first fixed volume well or reservoir 32 and a second fixed volume reservoir 34, also formed in the raised platforms 29. Recessed areas are formed between each of the raised platforms 29 and define air gaps 31. The illustrated electrodes 30A and 30B are planar and are flush with the outer surface of the platforms 29 as shown in Figs. 3A and 3D. The edges of the platforms 29 may be rounded for comfort when worn and pressed into the skin of the user.

During use of the biofluid computation measurement device 10, the wearer may sweat on and off over the course of several hours. The rapidity of the change in electrical signal correlating to when the wearer sweats and then stops sweating is affected by several parameters. One important factor is how quickly the sweat can dry between the skin-contacting electrode pairs 30A and 30B. The air gaps 31 between the platforms 29 allow the flow of air to quickly dry sweat within the air gaps 31.

The first fixed volume reservoir 32 includes a pair of electrodes 36 that do not make contact with skin and only make contact with perfused sweat when the reservoir 32 fills. A fluid conduit 38 may be formed in the first fixed volume reservoir 32 and is connected to an opening (not shown) in an edge of the electrode substrate 16. The second fixed volume reservoir 34 also includes a pair of the electrodes 36 that do not make contact with skin and only make contact with perfused sweat when the reservoir 34 fills. The second fixed volume reservoir 34 also includes a lateral fluid flow channel 40 formed in a wall thereof. Advantageously, the first fixed volume reservoir 32 and the second fixed volume reservoir 34 are open and easy to clean, thus allowing the electrode substrate 16 to be re-used by simply rinsing with water.

The second side 28 of the electrode substrate 16 includes a plurality of electrical traces 44. Vias 46 connect the electrodes 30A, 30B, and 36 to the traces 44 and are filled with metal and epoxy. Contact pads 47 are formed on the second side 28 and are configured for electrical connection to the spring-loaded pins of the spring connector 42 of the electronic module 12.

In an embodiment of the electrode substrate 16 having a PCB (not shown) mounted within the body 24, the PCB has the plurality of electrical traces 44 and other electrical components, such as a resistor 49, formed thereon, and the PCB electrically connects the electrodes 30A, 30B, and 36 to the contact pads 47. The contact pads 47 also connect to features such as the resistor 49, shown in the bottom center of the contact pads in Fig. 3C.

Resistors, such as the resistor 49, may serve as an identifier of the electrode substrate 16 where the electronic module 12 recognizes a specific resistance range as an identifier of the attachment in this case the EDA band when it is attached to the module. In addition, the resistor 49 may be a thermistor to measure temperature close to the skin or it may be a resistivity humidity sensor (electrical conductivity sensor) that can measure humidity close to skin. Alternatively, the resistor 49 may be any electrical resistance device, including, but not limited to a carbon based resistor and a thin film resistor. The resistor 49 may operate independently of temperature and humidity. Alternatively, the resistor 49 may be a type of resistor that is responsive to temperature and humidity. The resistor 49 may be comprised of multiple resistors combined in series and/or parallel, any or all of which may be responsive to temperature and/or humidity.

The electrode substrates disclosed herein may be formed from a variety of materials. For example, in one embodiment, the electrode substrate 16, shown in Figs. 3A through 3D may be formed from polymer, steel, and fiberglass, thus providing sub-components that can be quickly and inexpensively fabricated using mass-manufacturing processes. For example, the PCB (not shown) may be formed from fiberglass, such as FR4, or other composite material. The electrodes 30A, 30B, and 36 may be formed from steel, stainless steel, and machined surgical steel, including, but not limited to, 316, 420, and 440 stainless steel and soldered to the PCB. The body 24 may be from a rigid polymer, including, but not limited to, glass-filled nylon and ABS.

In another embodiment, the electrode substrate 16, shown in Figs. 3A through 3D may be formed from ceramic including, but not limited to, alumina and alumina composites that may be dry-pressed or sintered. This embodiment, formed from ceramic, provides for a functional, durable, aesthetically pleasing substrate that may be mass-manufactured and easily assembled into a larger assembly. The ceramic material has excellent chemical resistance, low dielectric loss, high strength, and is easy to clean. Additionally, the high temperature properties of the ceramic material allow for various metallization manufacturing techniques, including electroplating, that allow for flush electrodes with no reveal between the metal and the ceramic substrate.

The metallized electrodes are formed by electroplating gold to the ceramic surface of the raised platforms 29. Electrode and trace impedance is well controlled by the plating process and desired electrical impedance is easily achieved. Unlike the embodiment described above that uses steel electrodes, the gold electrodes of this embodiment have less charge polarization at the skin-electrode interface, as the half-cell potential of gold is lower than that of steel.

In yet another embodiment, the electrode substrate 16 shown in Figs. 3A through 3D may be formed from injection molded plastic such as glass filled nylon. Injection molded plastic serves as a very low cost, mass-producible material. The electrodes and traces may be formed in a manner similar to that of the ceramic electrode substrate 16, wherein metal is electroplated or applied through vapor deposition.

The electrode substrates described herein, including the electrode substrate 16 and the electrode substrates 48, 68, and 96 described below, are each configured to determine sweat volumetric rate and to measure electrolyte levels. Each electrode substrate has multiple pairs of skin-facing electrodes having different pitches, and can excite with different frequencies, including, but not limited to, frequencies within the range of about 100 Hz to about 10 kHz. Alternatively, the embodiments of the electrode substrates described herein may excite with frequencies up to about 100kHz. The skin-facing electrodes are configured to measure impedance of the skin tissue and any sweat that may form on the surface of the skin tissue. Thus, different aspects of sweat characteristics, such as when the sweat glands are about to release sweat to the surface, when sweat has perfused to the surface, and when sweat has dried from the surface, may be ascertained. Sequentially exciting the skin-facing electrodes prevents cross-talk and improves signal integrity. Having at least one fixed-volume reservoir allows for electrolyte measurements and can also provide rudimentary sweat volumetric rate measurements. Furthermore, having additional fixed-volume reservoirs of different sizes allows for additional rate measurements.

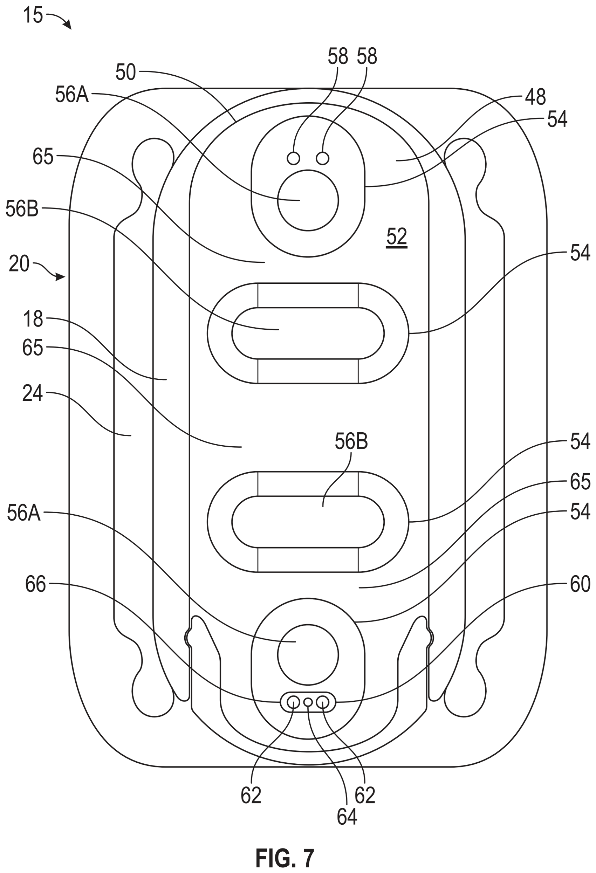

A second embodiment of the electrode substrate 48 is illustrated in Fig. 7 and shown mounted within the substrate housing portion 18 of the clip 20. The electrode substrate 48 includes a body 50 having a first or skin-facing side 52 and a second side (not shown) that faces away from the skin. The skin-facing side 52 includes raised platforms 54 upon which skin-contacting electrodes 56A and 56B are formed, i.e., the platforms 54 extend outwardly from the body 50 of the electrode substrate 48.

The electrodes 56B are configured to measure skin conductance, and the electrodes 56A are configured to measure conductance over a longer distance. One of the platforms 54 (the uppermost platform when viewing Fig. 7) includes a small pair of electrodes 58 configured to detect when the electrode substrate 48 is in contact with skin. The electrodes 58 have smaller pitch and a smaller surface area than the pairs of electrodes 56A and 56B, and are configured to measure the impedance of the top layers of the wearer's skin.

One of the platforms 54 (the lowermost platform when viewing Fig. 7) has a fixed volume reservoir 60 formed therein. A pair of electrodes 62 are formed in a recessed surface of the reservoir 60 and are configured to measure conductance in a known volume of sweat inside the reservoir 60. An outlet hole 64 is formed in the recessed surface of the reservoir 60 between the electrodes 62 and is configured to allow the reservoir 60 to fill completely before allowing excess sweat to exit.

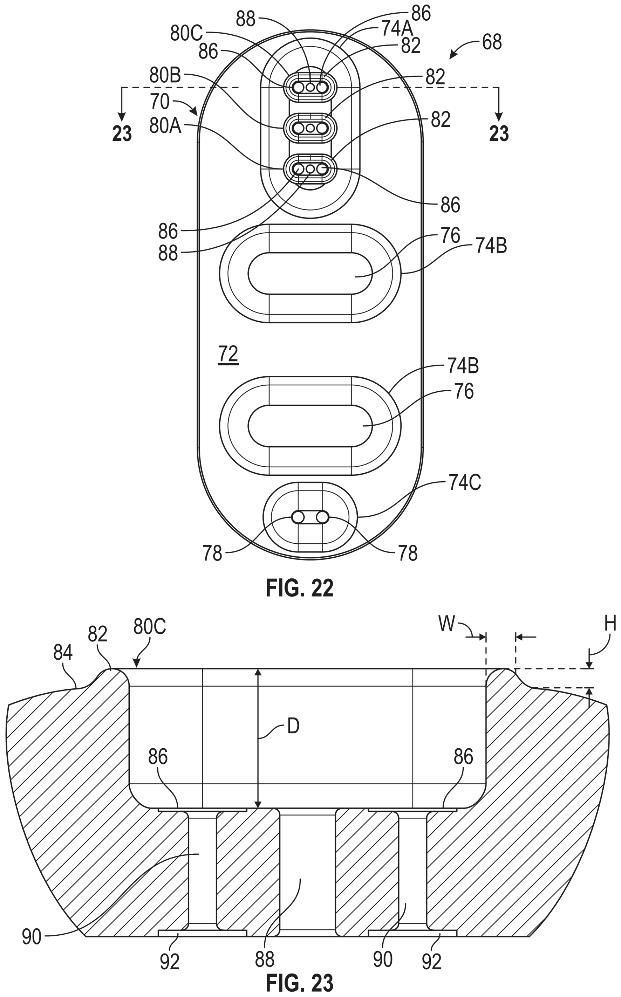

The electrodes 62 do not contact the skin, but make contact only with biofluid. A seal is formed by an outer edge or outwardly extending lip 66 of the reservoir 60, as shown in Fig. 23, pressing against the skin surface, allowing the reservoir 60 to fill with sweat. The lip 66 thus defines an inlet port of the reservoir 60. The outlet hole 64 is sized to be sufficiently small so that surface tension of the biofluid in the reservoir 60 prevents sweat from leaving the reservoir 60 until the reservoir 60 has completely filled. The fixed volume reservoir 60 thus performs two important functions. First, the fixed volume reservoir 60 enables a conductivity measurement of a known volume of biofluid, such as sweat. Secondly, the fixed volume reservoir 60 works in conjunction with the skin-facing electrodes 56A and 56B. The time required to fill the reservoir 60 may also be calculated to determine a sweat perfusion rate. It will be appreciated that at high enough excitation frequencies, the effective sweat volume within the reservoir 60 is limited by the size of the electromagnetic fringe field and becomes independent of reservoir geometry, such as reservoir depth.

The lip 66 of the reservoir 60 may be rounded for comfort when the electrode substrate 48 is worn and pressed into the skin of the user. Alternatively, the lip 66 of the reservoir 60 may also be identical to the lip 82 shown in Fig. 23, such that the lip 66 has sufficient width to form a seal and the edge is rounded for comfort when the electrode substrate 48 is worn and pressed into the skin of the user. As shown in Fig. 23, the lip 82 extends around an edge of the reservoir 80A, and presses into the user's skin to create an improved seal. The lip 82 has a height H of about 0.25 mm from an initial sealing surface 84, and has a width W of about 0.5 mm. Alternatively, the lip 82 may have a height H within the range of about 0.125 mm to about 0.375 mm, and a width W within the range of about 0.375 mm to about 0.625 mm.

Recessed areas are formed between each of the raised platforms 54 and define air gaps 65. The illustrated electrodes 56A, 56B, 58, and 62 are planar and are flush with the outer surface of the platforms 54. The edges of the platforms 54 may be rounded for comfort when worn and pressed into the skin of the user.

Fig. 22 is an enlarged bottom plan view of a third embodiment of the electrode substrate 68, and is shown without the clip 20 for clarity. The electrode substrate 68 includes a body 70 having a first or skin-facing side 72 and a second side (not shown) that faces away from the skin. The skin-facing side 72 includes raised platforms 74A, 74B, and 74C, i.e., the platforms 74A, 74B, and 74C extend outwardly from the body 70 of the electrode substrate 68 upon which skin-contacting electrodes 76 and 78 are formed, and within which fixed volume reservoirs of increasing volume 80A, 80B, and 80C are formed.

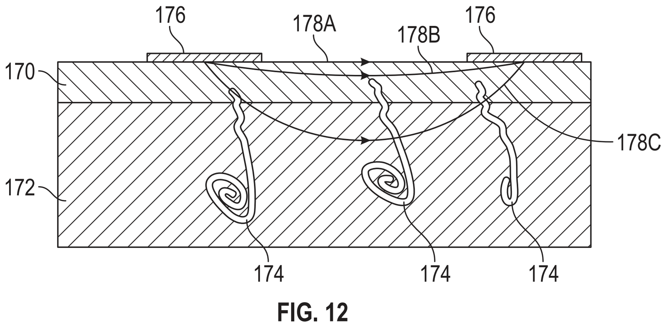

The illustrated electrodes 76 are planar and are flush with the outer surface of the platforms 74B. Similarly, the electrodes 78 are planar and are flush with the outer surface of the platform 74C. The edges of the platforms 74A, 74B, and 74C may be rounded for comfort when worn and pressed into the skin of the user. The electrodes 76 are configured to measure the impedance of the top layers of the wearer's tissue or skin, as shown in Fig. 12, and described herein above.

The platform 74A (the uppermost platform when viewing Fig. 22) includes three fixed volume reservoirs of increasing volume 80A, 80B, and 80C formed therein.

Each of the reservoirs 80A, 80B, and 80C has a lip 82 that extends around an edge of the reservoirs 80A, 80B, and 80C, and presses into the user's skin to create an improved seal. The lip 82 has sufficient width to form a seal and is rounded for comfort when the electrode substrate 68 is worn and pressed into the skin of the user. As shown in Fig. 23, the lip 82 extends around an edge of the reservoirs 80A, 80B, and 80C, and presses into the user's skin to create an improved seal. The lip 82 has a height H of about 0.25 mm from an initial sealing surface 84, and has a width W of about 0.5 mm. Alternatively, the lip 82 may have a height H within the range of about 0.125 mm to about 0.375 mm, and a width W within the range of about 0.375 mm to about 0.625 mm.

It will be understood that each of the reservoirs 80A, 80B, and 80C has a different depth D. The reservoir 80C illustrated in Fig. 23 has a depth D of about 2 mm. The reservoirs 80B and 80A have depths of about 1.5 mm and 1 mm, respectively. The electrodes 78 on the raised platform 74C define a zero-depth reservoir.

Each of the reservoirs 80A, 80B, and 80C include a pair of electrodes 86 that do not make direct, physical contact with skin and only make contact with perfused sweat when the reservoirs 80A, 80B, and 80C fill. A fluid outlet 88 may be formed in the reservoirs 80A, 80B, and 80C and is connected to an opening (not shown) in an edge of the electrode substrate 68. Electrical conduits 90 connect the electrodes 86 to electrical traces 92.

As the user begins to sweat, the sweat is first sensed by the zero-depth electrodes 78. As the user continues to sweat, the shallowest depth reservoir 80A fills first, followed by the reservoirs 80B and 80C. Advantageously, the electrode substrate 68 is configured to measure sweat at different locations, i.e., at the zero-depth electrodes 78, and within the reservoirs 80A, 80B, and 80C. The electrode substrate 68 can further measure the sweat rate change over time.

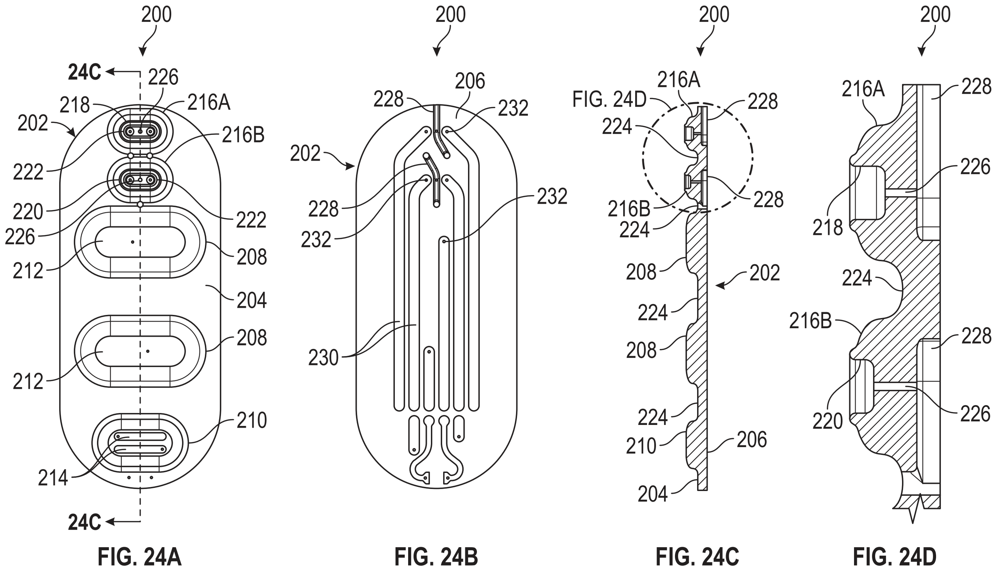

The fourth embodiment of the electrode substrate 200, shown in Figs. 24A through 24D includes a body 202 having a first or skin-facing side 204 and a second side 206 that faces away from the skin. The substrate may be formed out of sintered ceramic such as, but not limited to, alumina. Alternatively, the substrate may be formed from injection molded polymers. Electrodes and traces may be metallized using techniques such as but not limited to electroplating or vapor deposition. If a PCB is embedded inside, it may serve as the traces to connect the electrodes on the skin-facing side to the contact pads on the second side. In that case, electrodes may be formed using stamped or machine metal as another method to interface to skin.

The skin-facing side 204 includes raised platforms 208 and 210, upon which pill shaped, skin-contacting electrodes 212 and 214 are formed, i.e., the platforms 208 and 210 extend outwardly from the body 202 of the electrode substrate 200. The illustrated electrodes 212 and 214 are planar and are flush with the outer surface of the platforms 208 and 210 as shown in Figs. 24A and 24C. The electrodes 212 are configured to measure the impedance of the top layers of the wearer's tissue or skin, as shown in Fig. 12, and described herein above. The illustrated electrodes 214 have smaller pitch and a smaller surface area than the electrodes 212. Additionally, the electrodes 214 on the raised platform 210 define a zero-depth reservoir.

The skin-facing side 204 further includes two additional raised platforms 216A and 216B. The platform 216A has a first fixed volume reservoir 218 having a first depth of about 1.25mm Similarly, the platform 216B has a second fixed volume reservoir 220 having a second depth of about 0.75mm. A pair of circular electrodes 222 are formed in a lower surface of the first and second reservoirs 218 and 220, respectively.

The edges of the raised platforms 208, 210, 216A, and 216B may be rounded for comfort when worn and pressed into the skin of the user. Recessed areas are formed between each of the raised platforms 208, 210, 216A, and 216B and define air gaps 224. The air gaps 224 between the platforms 208, 210, 216A, and 216B allow the flow of air to quickly dry sweat within the air gaps 224.

The electrodes 222 within the first and second reservoirs 218 and 220 do not make direct, physical contact with skin and only make contact with perfused sweat when the reservoirs 218 and 220 fill. A fluid conduit 226 may be formed in each of the first and second reservoirs 218 and 220 and is connected to a channel 228 in the second side 206 of the electrode substrate 200.

As the user begins to sweat, the sweat is first sensed by the zero-depth electrodes 214. As the user continues to sweat, the shallowest depth reservoir 220 fills first, followed by the reservoir 218. Advantageously, the electrode substrate 200 is configured to measure sweat at different locations, i.e., at the zero-depth electrodes 214, and within the reservoirs 218 and 220. The electrode substrate 200 can further measure the sweat rate change over time.

Advantageously, the first and second reservoirs 218 and 220 are open and easy to clean, thus allowing the electrode substrate 200 to be re-used by simply rinsing with water.

The second side 206 of the electrode substrate 200 includes a plurality of electrical traces 230. Vias 232 connect the electrodes 212, 214, and 222 to the traces 230 and are filled with metal and epoxy. Portions of the traces 230 are configured for electrical connection to the spring-loaded pins of the spring connector 42 of the electronic module 12.

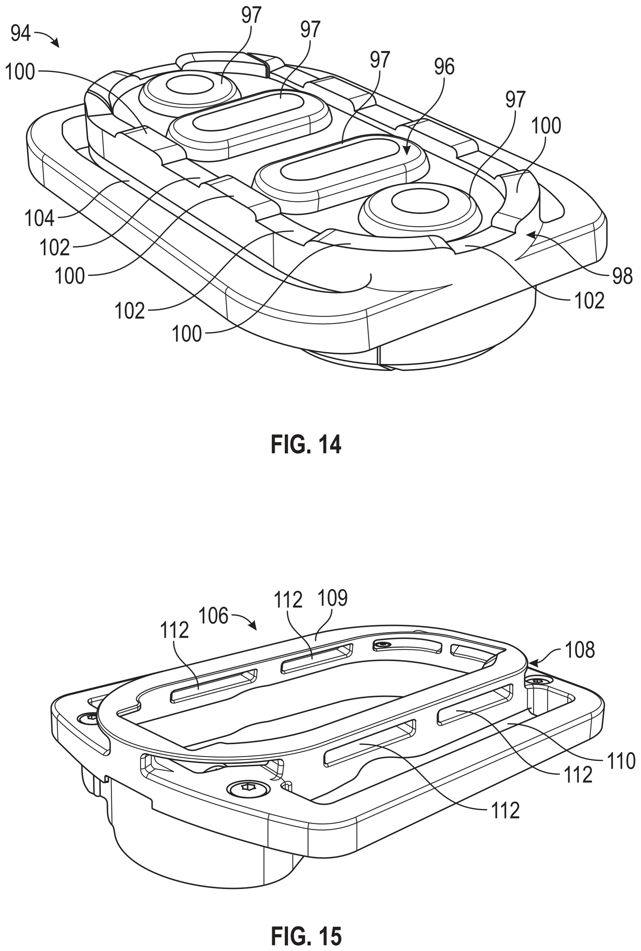

Fig. 14 is a perspective view of a first alternate embodiment of the clip 94 shown with an electrode substrate 96 formed thereto. The electrode substrate 96 includes a plurality of skin-facing raised electrode platforms 97. The clip 94 is similar to the clip 20 and includes a substrate housing portion 98 and slots 104 for the attachment of the compression strap 22. A wall of the substrate housing portion 98 includes a plurality of castellations 100 defining airflow passages 102 therebetween. Advantageously, air can circulate through the opened airflow passages 102 in the wall of the substrate housing portion 98 and between the skin-facing raised electrode platforms 97. The combination of the raised electrode platforms 97 and the venting in the wall of the substrate housing portion 98 created by the airflow passages 102, provides improved air flow and an improved ability to dry the skin. When sweating halts, the biofluid computation measurement device 10 can quickly detect a change in signal and is not deceived by remnant sweat on the skin.

Fig. 15 is a perspective view of a second alternate embodiment of the clip 106 shown without an electrode substrate for clarity. The clip 106 is similar to the clip 94 and includes a substrate housing portion 108 having a continuous skin facing surface 109 and slots 110 for the attachment of the compression strap 22. A wall of the substrate housing portion 108 includes a plurality of slots defining airflow passages 112. Like the clip 94, air can advantageously circulate through the airflow passages 112 in the wall of the substrate housing portion 108 and between the skin-facing raised electrode platforms, such as the skin-facing raised electrode platforms 97. Advantageously, when the skin facing surface 109 of the clip 106 is pressed into the skin, the airflow passages 112 are spaced apart from the skin, thus preventing the skin from covering the airflow passages 112. The combination of the raised electrode platforms 97 and the venting in the wall of the substrate housing portion 108 created by the airflow passages 112, provides improved air flow and an improved ability to dry the skin. When sweating halts, the biofluid computation measurement device 10 can quickly detect a change in signal and is not deceived by remnant sweat on the skin.

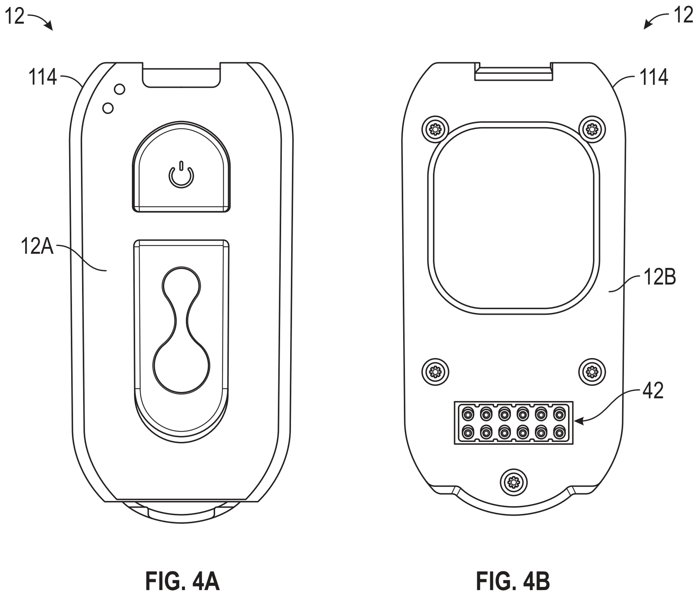

Fig. 6 is an exploded perspective view of the reusable electronic module 12 shown in Figs. 1, 4A, and 4B.

The electronic module 12 includes a housing 114 having an upper portion 114A, a lower portion 114B, and gaskets 118A and 188B. The housing 114 and the gaskets 118A and 118B provide electrical protection while blocking the ingress of water and particles, such as dust and dirt. Large, easy to press buttons 116 are contained within, and extend partially outwardly through, the upper portion 114A of the housing 114, and allow user input to the electronic module 12.

The electronic module 12 further includes a PCB 120 having electronic components mounted thereto, including but not limited to, the spring connector 42, a transimpedance amplifier 122 for measuring currents conducted through a fluidic patch or on the skin, an accelerometer 124 for measuring movement of the wearer, dual temperature sensors 126 and 126' for measuring near-skin temperature and a temperature gradient related to heat flux, a microcontroller 127, and a haptic motor 128 for providing tactile feedback to users.

Advantageously, the electronic module 12 includes a haptic alert and/or an alarm that activates when the sensor readings from the electrode substrate, such as the electrode substrate 16, exceed a pre-determined threshold measurement including, but not limited to, sweat volume, sweat deficit, defined as sweat volume lost minus fluid intake, and core-temperature. Additionally, the measurements taken by the electrode substrate 16 are wirelessly transmitted to the smart device.

Figs. 5A and 5B are top and bottom plan views, respectively, of a disposable, one-time use fluidic patch 130. The fluidic patch 130 is a one-time use microfluidic patch that captures sweat and adheres to skin with adhesive. The fluidic patch 130 has a first or skin-facing side 132 and a second side 135 that faces away from the skin. Examples of suitable one-time use microfluidic patches are disclosed in US Patent No. 11,559,225, the disclosure of which is incorporated herein in its entirety.

The illustrated one-time use fluidic patch 130 includes an adhesive, such as a striated patterned adhesive, on the surface of the skin-facing side 132. A microfluidic channel 138 is formed within the fluidic patch 130 and has an inlet port 134 and an outlet port 133. A seal around the microfluidic inlet port 134 is formed by the adhesive that attaches to skin and creates a water-tight seal to the skin. Perfused sweat from the wearer enters the microfluidic channel 138 through the inlet port 134 and exits through the outlet port 133. A plurality of electrodes 136 are disposed within the microfluidic channel 138. The plurality of electrodes 136 are in contact with the captured perfused sweat within the microfluidic channel 138, and are configured to measure fluid volume and fluid conductivity. Thus, the plurality of electrodes 136 are not exposed to the outside environment, but are only exposed to the captured perfused sweat in the microfluidic channel 138. As perfused sweat fills the microfluidic channel 138, the electrodes 136 sequentially make contact with the electrically-conductive sweat, and very small changes in sweat volume may easily be detected. The second side 135 of the fluidic patch 130 is configured to have the re-usable electronic module 12 mounted thereto and includes contact pads 137 configured for electrical connection to the spring-loaded pins of the spring connector 42 of the electronic module 12. When combined, the fluidic patch 130 and the re-usable electronic module 12 define a second embodiment of the biofluid computation measurement system.

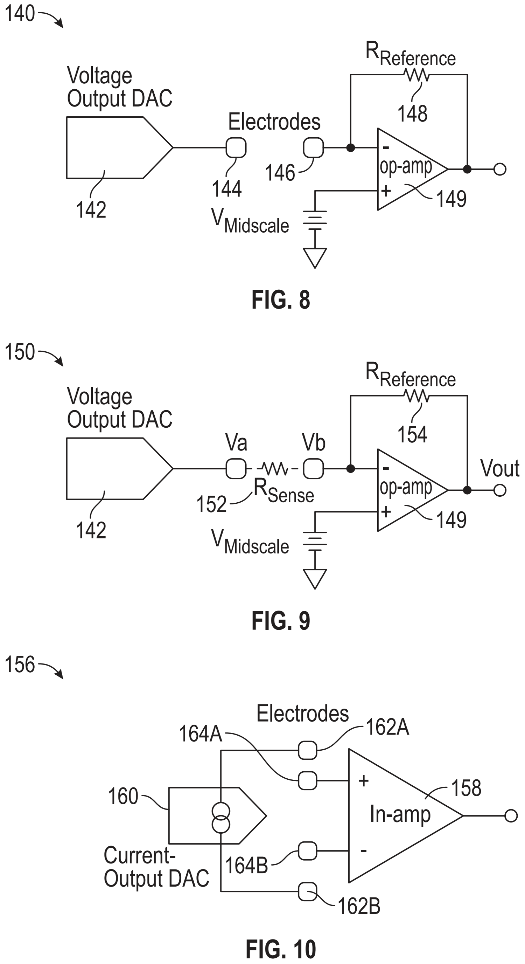

Fig. 8 is a schematic diagram of a two point measurement circuit 140 using a transimpedance amplifier topology. The circuit 140 includes a digital-to-analog converter (DAC) 142 to generate a known voltage, a first electrode 144 for applying the voltage generated to the substance to be measured, e.g., skin or sweat in a fluidic channel, a second electrode 146 connected to the negative terminal of an operational amplifier (op amp) to allow current to pass through, and a reference resistor 148 that generates a voltage at the circuit output proportional to the current passed between the electrodes.

In the circuit 140, the DAC 142 provides a time-varying voltage to the first electrode 144, wherein the signal delivered to the first electrode 144 is referred to as an excitation signal. The second electrode 146 is connected to the negative terminal of an operational amplifier (op-amp) 149 configured in a transimpedance circuit topology. A mid-scale DC bias voltage, VMidscale, is set by a voltage reference on the non-inverting terminal of the op-amp 149. This mid-scale voltage serves as the "ground" for the first electrode 144. A voltage drop across the first and second electrodes 144 and 146 generates a current.

Fig. 9 is a schematic diagram of a transimpedance amplifier circuit 150 illustrating how the resistance between electrodes correspond to the voltage of the transimpedance amplifier.

In the circuit 150, the current flows through a material, represented electrically as a resistance $R_{Sense}$ 152, positioned between two electrodes $V_a$ and $V_b$. $R_{Sense}$ 152, may include material such as tissue, for example skin, or fluid, for example sweat. The current then enters the transimpedance amplifier circuit 150 and then the current flows into the reference resistor, $R_{Reference}$ 154.

The resistance between the electrodes $V_a$ and $V_b$ will correspond to the voltage of the transimpedance amplifier as follows:

$$R_{Sense} = ((V_a - V_b) * R_{Reference})/(V_b - V_{out}),$$

Wherein the voltage of the op-amp 149 is inversely proportional to the resistance between the electrodes, RSense, and scaled by the feedback reference resistor, $R_{Reference}$ 154. Example elements that may be measured across the electrodes $V_a$ and $V_b$ include, but are not limited to, sweat and tissue impedance. The DAC 142 outputs waveforms of varying morphology and frequency. In this embodiment, the DAC 142 outputs a sinusoid of fixed voltage magnitude and a set of targeted frequencies that have been empirically determined for specific conductance signatures corresponding to specific phases of sweat production.

Fig. 10 is a schematic diagram of a conductance measurement circuit 156 having a four point measurement circuit topology.

The illustrated conductance measurement circuit 156 includes an instrumentation amplifier (in-amp) 158 to measure the voltage across the electrode in the circuit 156 when an excitation source generates current across the electrodes in the circuit 156, or separate electrodes (not shown) nearby and in contact with the same target material. For example, Fig. 10 illustrates such a circuit 156 in a four-point measurement configuration. The circuit 156 includes a current output digital to analog converter (DAC) 160 that sends an excitation current from a first excitation or force electrode 162A of a pair of excitation or force electrodes to a second excitation or force electrode 162B. A pair of sense electrodes 164A and 164B are nested between the force electrodes 162A and 162B. The sense electrodes 164A and 164B are measured by the in-amp 158. The current output DAC 160 outputs a time varying current. The in-amp 158 measures the change in voltage between the sense electrodes 164A and 164B. The excitation current from the force electrodes 162A and 162B passes through the region across the sense electrodes 164A and 164B. The resistance between electrodes 164A and 164B is indicated by the voltage measured by the in-amp 158 divided by the current supplied by the current output DAC 160 and passed through electrodes 162A and 162B.

Examples of elements that may be measured across the sense electrodes 164A and 164B include, but are not limited to, sweat and skin tissue. One of the benefits of a 4-point measurement is that it mitigates the effect of overpotential voltage build up on the electrodes 162A, 162B, 164A, and 164B due to charge polarization that would affect the measurement reading. This feature allows the use of metal electrodes, which are easier to fabricate, to measure the impedance of fluid and/or tissues. This topology can be converted to a two-point instrumentation amplifier circuit topology by fabricating the electrodes 162A and 164A as one single electrode, and the electrodes 162B and 164B as one single electrode.

The current output DAC 160 is capable of outputting waveforms of various current magnitudes, morphologies, and frequencies. In one embodiment, the current output DAC 160 generates a fixed magnitude sinusoid current at specific frequencies that are able to isolate certain conductance signatures corresponding to specific stages of sweat production. Circuits 140, 150 and 156 may be constructed out of discrete active components. However, all three topologies may be designed into an application specific integrated circuit (ASIC) for unit cost reduction, size reduction, and system performance improvements through improved tolerance matching.

The circuit topologies of the circuits 140, 150, and 156 in Figs. 8, 9, and 10, respectively, all measure conductance, and may be used to measure the conductance of different materials. In one embodiment, the circuit 140, 150, and 156 may be connected to an electrode pair in contact with skin tissue, such as the inner, skin-facing, electrodes 56B shown in Fig. 7. In another embodiment, the circuit 140, 150, and 156 may measure a material that is not skin tissue, such as when the circuit 140, 150, and 156 is connected to the fluid-facing electrodes 62 in the reservoir 60, shown in Fig. 7.

In another embodiment, several conductance measurements may be made to ascertain different characteristics of sweat production at any given time. For example, a conductance measurement of the very close-pitched, skin-facing electrodes 58, shown in Fig. 7, may be performed sequentially with a conductance measurement of the inner, skin-facing, electrode pair 56B, and sequentially with a conductance measurement of the fluid-facing electrodes 62 in the reservoir 60. The electrodes are all measured sequentially in quick succession, e.g., within milliseconds, and then the next sample for the same electrodes occur after a larger timeframe, e.g., within 10 seconds. Effectively at a physiological level, it appears that the collection of measurements sampled every 10 seconds is taken simultaneously, however, current only flows across one electrode pair at a time, thereby avoiding cross-talk.

Fig. 12 is a schematic cross-sectional view of human epidermis and dermis showing skin-facing electrodes in contact with the epidermis and current paths vs. skin depth for varying levels of excitation.

Fig. 12 shows the epidermis 170 above the dermis 172. Sweat glands 174 are interspersed within the dermis 172 and epidermis 170. Examples of skin-facing electrodes 176 are illustrated and shown in contact with the epidermis 170. The depth of the current that passes through skin is commensurate with the excitation frequency. For example, at low frequencies, the current path 178A is closer to the surface of the epidermis 170, and spreads deeper into the tissue at higher frequencies (see the arrows 178B and 178C). Generally, the electrical signal that corresponds to the top layer of the epidermis (stratum corneum) dominates when excitation is below 1kHz. In one embodiment, the conductance circuit generates several excitation frequencies spanning low-to-high to identify conductance changes in various depths of skin tissue as well as to detect the presence of a laminar fluid layer if sweat has formed across the epidermis surface.

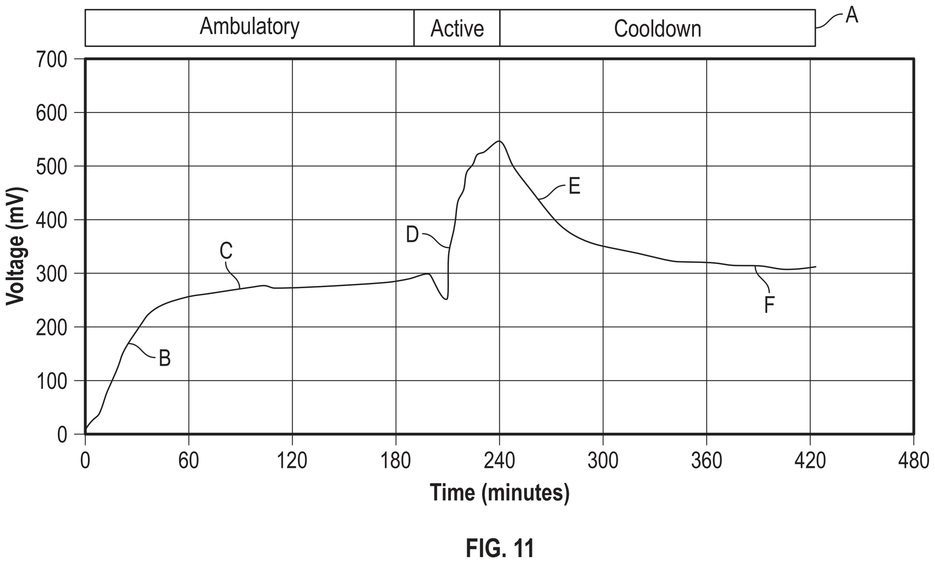

Sweat production and cessation trends are easily captured in the conductance waveforms of the skin-facing electrodes described herein. Fig. 11 is a graph of an electrical conductance signal from a skin-engaging electrode (mV) vs. time (minutes) for a subject during periods of ambulatory activity, active activity, and cooldown (A). The signal's response to the activity is dynamic, rising immediately after the device, such as the biofluid computation measurement device 10, is secured against the skin (B) until it reaches a baseline (C), then rising again in response to the skin's reaction to activity and the presence of sweat (D), and falling after the activity is completed and the presence of sweat is reduced (E), until a new post-activity baseline is reached (F).

As shown, the electrical signal from the skin-facing electrodes increases with sweat onset and reduces when sweating halts. The voltage signal in Fig.

11 corresponds to the output of the transimpedance amplifier circuit 150 illustrated in Fig. 9 and the inner skin-facing electrodes 56B in Fig. 7. The graph in Fig, 11 lists the subject's activity at the time of the measurement. After the subject secures the device to their arm (Fig. 11 B), they start performing low-intensity ambulatory tasks (Fig. 11 C). The corresponding signal for ambulatory movement shows low magnitude and will be referred to as a baseline. Eventually, the subject performs active exercise. The onset of sweat during activity is marked by a very quick, steep slope increase as indicated in Fig. 11 D. However, even at the onset of activity, the baseline remains low until sweat perfuses skin at Fig. 11 D. During the cooldown period when the subject halts exercise, the electrical signal falls (Fig. 11 E), and the signal ultimately falls to a level close to the ambulatory baseline magnitude, as shown at Fig. 11 F.

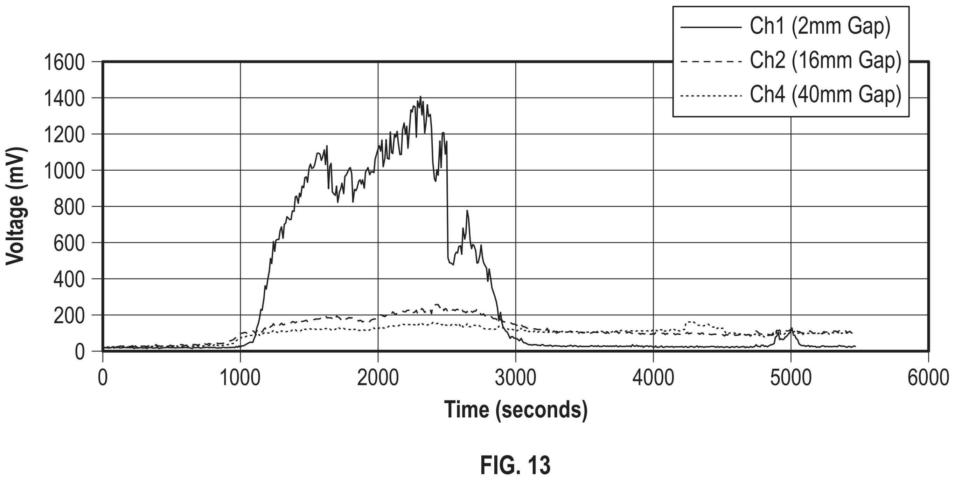

In one embodiment of the biofluid computation measurement device 10, a small skin-facing electrode pitch is used to isolate the onset of when a laminar fluid layer of sweat resides on the surface of the skin compared to when the sweat glands start sweat activity, but sweat has not yet appeared on the surface. Fig. 13 is a graph of an electrical conductance signal from a skin-engaging electrode (mV) at different electrode pitches vs. time (seconds). Specifically, plots of electrical conductance signals corresponding to 2 mm, 16 mm, and 40 mm electrode pitches (gaps between electrodes) are shown. In this embodiment, the 2 mm gap electrodes are a much smaller gap than the 16 mm and 40 mm electrode gaps, and therefore the voltage measurements for the 2 mm gap electrodes are expected to be much higher than for the 16 mm and 40 mm electrodes. Fig. 13 shows the conductance signal for three skin-facing electrode pitch distances for an excitation of 1kHz on a human subject. In the graph, the voltage conductance signal response for very small pitch skin-facing electrodes having a 2mm gap and 1kHz excitation has a steeper slope relative to the other pitch response curves and that may be exploited as an easily automatable reference point indicating sweat perfusion on the surface of the skin and a comparison time-stamp to the fill-times in wells 32, 34, 60, 80A, 80B, and 80C.

In another embodiment of the biofluid computation measurement device 10, characteristics of sweat activity before sweat has perfused to the surface of the skin and after sweat has perfused to the surface of the skin may be detected by using a combination of electrodes with at least two pitches, such as shown in Fig. 13, and varying excitation frequencies, as shown in Fig. 12, to provide insight as to the onset of sweat, halting of sweat and the sweat rate.

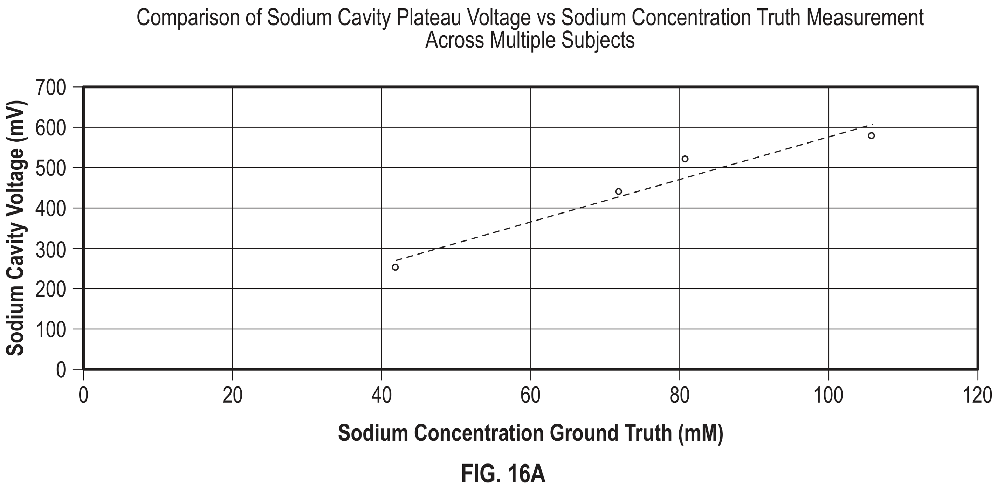

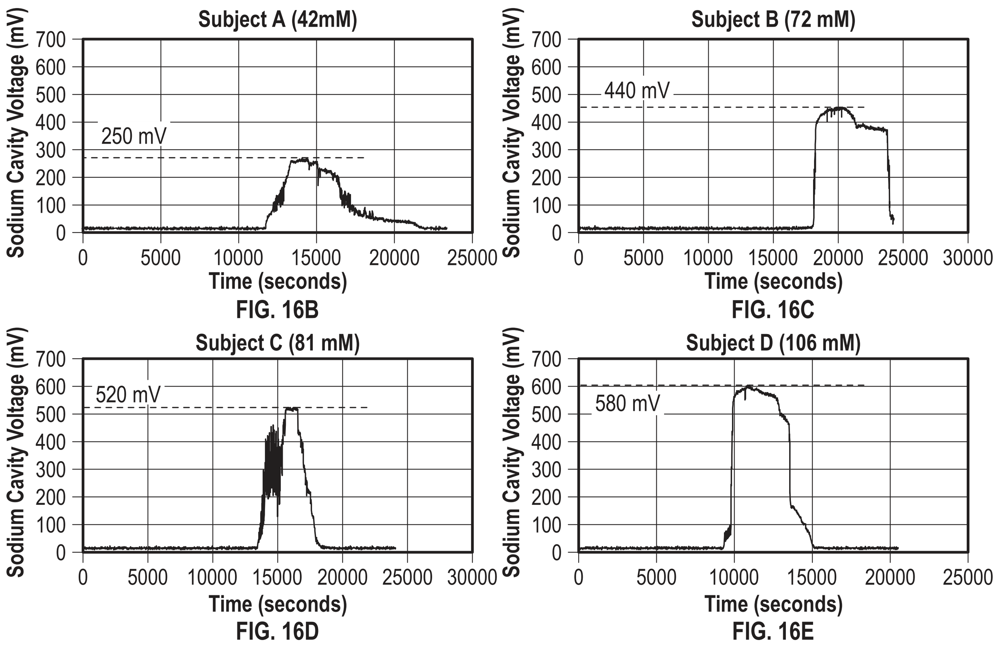

Fig. 16A is a graph comparing sodium cavity plateau voltage vs. measured sodium concentration across four unique subjects and is derived from the graphs shown in Figs. 16B through 16E. The correlation between a subject's sodium concentration and the measured voltage signal may be used in an algorithm to determine sodium concentration of an individual.

Figs. 16B through 16E are graphs of electrical conductance voltage of captured sweat in a cavity of known volume vs. time for four unique subjects with different sweat sodium concentration levels. The volume of sweat captured in the cavity is consistent across subjects, so measurements differ due to the difference in sodium concentrations of the captured sweat.

Sweat conductivity for subjects varies depending on electrolyte content and total volume of sweat present. A reservoir of known volume, including, for example, the reservoirs 32, 34, and 60, constrains the sweat to a known volume when the reservoir is filled. The electrical conductivity of the known volume can then be assessed, and any measurement differences attributed to differences in electrolyte levels. It takes some amount of time for the reservoir to fill with perfused sweat as the subject exercises. The graphs in Figs. 16B through 16E show the electrical conductance voltage vs. time (in samples) for four unique subjects performing some physical exertion. Each waveform shows a peak value where the reservoir has completely filled and the signal plateaus. This maximum electrical value is captured and compared to a lookup table or mathematical transfer function of correlated electrolyte concentrations, primarily dominated by Na+ and Cl-, to electrical conductivity values.

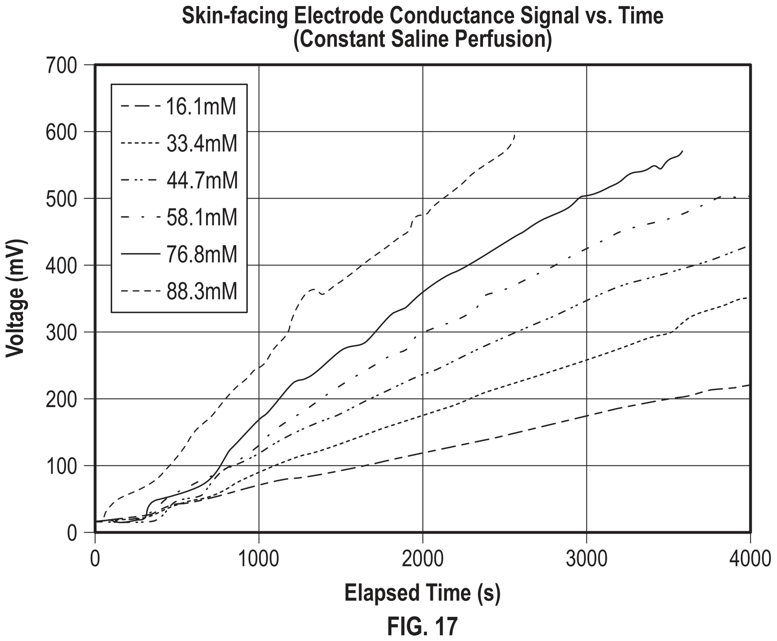

The skin-side electrode conductivity signal can reveal different characteristics of sweat based on frequency and electrode pitch. If the signal response that targets the surface of the skin covered with perfused sweat is assessed, the rate of signal growth may be determined by the electrolyte concentration and the amount of fluid present. Fig. 17 is a graph of an electrical conductance signal of artificial skin being perfused at constant rate with sodium saline solutions of varying concentrations. Each trace in the graph shown in Fig. 17 represents a specific molar concentration of sodium solution. The graph shows increasing rates of signal growth for higher sodium concentrations when perfused at the same rate compared to lower sodium concentrations. The inverse can be computed to assess perfusion rate when sodium concentration is known.

In one embodiment, measurements using the skin-facing electrodes and fluid-facing electrodes described herein may be enhanced by correlating the aforementioned measurements with motion measurements from the electronic module's 12 accelerometer 124 and skin-temperature data from the on-board temperature sensors 126. Motion and skin-temperature data can provide additional context to sweat rate values based on skin-facing electrodes and the fill volume of a fixed-volume reservoir.

While the biofluid computation measurement device 10, including the re-usable electronic module 12 mounted to the re-usable EDA band assembly 14 having the electrode substrate 16 mounted within the substrate housing portion 18 of the clip 20 includes features that can determine sweat rate over time, the lack of a deep fluidic channel with embedded electrodes in the channel limits the precision of the volume measurement over a long session. Advantageously, both the fluidic patch 130 with the attached re-usable electronic module 12, and the biofluid computation measurement device 10, may be used to measure unique aspects of sweat production, and they may be used sequentially in time.

For example, the fluidic patch 130 has the extensive microfluidic channel 138 and can characterize sweat rate changes over several hours as the user's motion and skin-temperature rise and fall as exertion, and environmental temperatures change throughout the day. Accurate electrolyte measurements are simultaneously and easily made despite the changing conditions because sweat is captured in the fluidic patch 130. In contrast, the biofluid computation measurement device 10 has limited fluidic capture capability because it is re-usable and therefore needs to be easily cleanable.

Thus, some limitations of the biofluid computation measurement device 10 may be overcome by using the fluidic patch 130 for a period of time to capture certain characteristics of a user's sweat response. The resulting profile may then be used to refine estimates of sweat loss when using biofluid computation measurement device 10. The profile created is unique to the user, and can be stored on the electronic module 12 or in the cloud. It will be understood that the electronic module 12 used with the fluidic patch 130 may be transferred to the biofluid computation measurement device 10. The profile may then be retrieved and applied when the biofluid computation measurement device 10 makes a measurement. The characteristic sweat profile can provide additional information when computing sweat rate that relies on skin-facing electrodes, such as the electrodes 30A, 30B, 36, 56A, 56B, 58, and 62, conductance, and ancillary measurements such as skin temperature and movement. For example, one user may sweat at high rates when their skin temperature is high, regardless of their movement. Another user may sweat at lower rates, but only when moving vigorously. Such profiles can inform predictions of sweat loss using the biofluid computation measurement device 10.

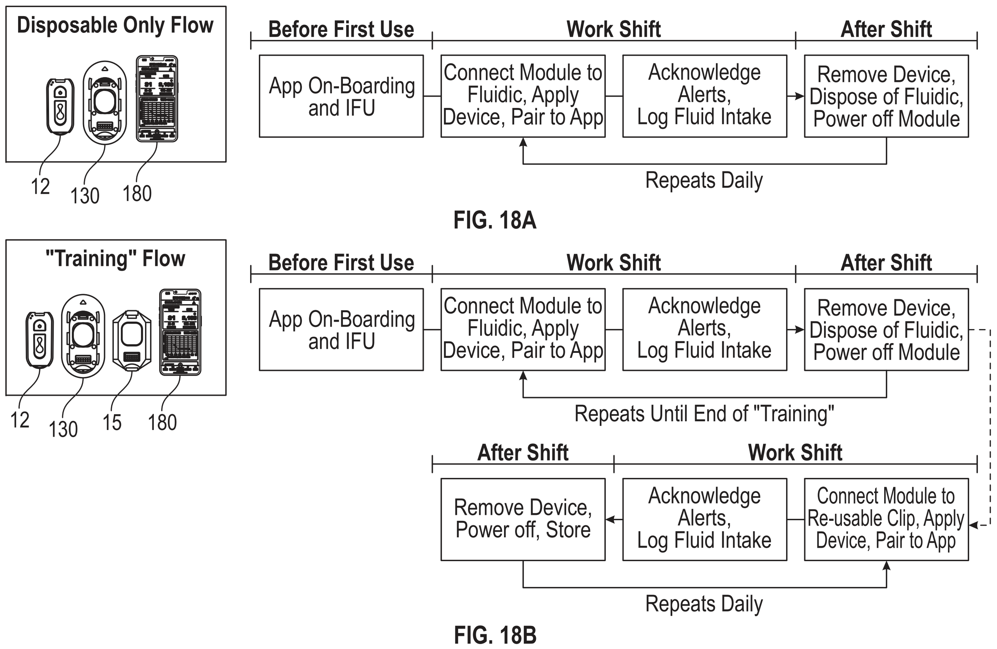

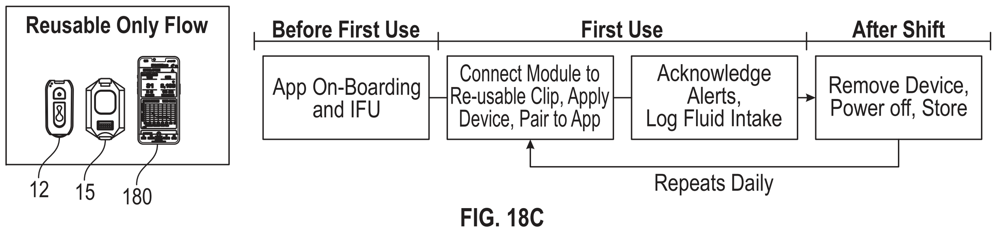

Advantageously, a user may use one or both of the fluidic patch 130 and the biofluid computation measurement device 10 in any one of three ways as shown in Figs. 18A, 18B, and 18C.

Fig. 18A is a flow chart illustrating the process of using only the disposable fluidic patch 130 with the re-usable electronic module 12 and a smartphone 180.

In the flow chart illustrated in Fig. 18A, the user only uses the disposable fluidic patch 130 with the re-usable electronic module 12 and the smartphone 180. As shown, the user first follows the instructions for use (IFU) provided on the smartphone's mobile app, described below, and enters any requested information. The user then connects the electronic module 12 to the fluidic patch 130, applies the fluidic patch 130 to the skin, and pairs the electronic module 12 to the mobile app. During use, the user also acknowledges any alerts noted on the mobile app and logs fluid intake into the mobile app. After use, the user powers off the electronic module 12, removes the fluidic patch 130, separates the fluidic patch 130 from the electronic module 12, and disposes the fluidic patch 130.

Fig. 18B is a flow chart illustrating the process of first using the disposable fluidic patch 130 with the electronic module 12 and the smartphone 180 to build a characteristic sweat profile, then, after a training period, the user switches to the re-usable biofluid computation measurement device 10.

In this embodiment, the one-time use fluidic patch 130 is applied to a wearer and used to measure the wearer's activity, temperature, sweat volume, and electrolyte captured levels to generate a characteristic sweat profile consisting of coefficients from the correlation of sweat loss for given activity, temperature, and time duration. The one-time use fluidic patch 130 is then disconnected from the re-usable electronic module 12, and re-usable electronic module 12 is attached to the wearable biofluid computation measurement device 10. The wearable biofluid computation measurement device 10 is then applied to the wearer, such that the skin-facing electrodes measure sweat onset, sweat rate, and electrolyte conductivity. The measurement computation from the wearable biofluid computation measurement device 10 may be augmented with the characteristic sweat profile developed from the one-time use fluidic patch 130.

As shown in the flow chart illustrated in Fig. 18B, the user first follows the IFU provided on the mobile app, described below, and enters any requested information. The user first uses the fluidic patch 130 to build a characteristic sweat profile. As in Fig. 18A, the user then connects the electronic module 12 to the fluidic patch 130, applies the fluidic patch 130 to the skin, and pairs the electronic module 12 to the mobile app. During use, the user also acknowledges any alerts noted on the mobile app and logs fluid intake into the mobile app. After use, the user powers off the electronic module 12, removes the fluidic patch 130, separates the fluidic patch 130 from the electronic module 12, and disposes the fluidic patch 130. This process is repeated until the designated duration of the training period has ended.

After the training period, which may last several days, has ended, the user then connects the electronic module 12 to clip assembly 15 and attaches the re-usable biofluid computation measurement device 10 to the body, such as an arm, using the compression strap 22. During use, the user again acknowledges any alerts noted on the mobile app and logs fluid intake into the mobile app. Sensors in the electronic module 12 are used to obtain an enhanced measurements that have improved precision. After use, the user powers off the electronic module 12, and removes and stores the re-usable biofluid computation measurement device 10 for later use.

Fig. 18C is a flow chart illustrating the process of using only the re-usable biofluid computation measurement device 10, including the electronic module 12 and the smartphone 180. In the flow chart illustrated in Fig. 18C, the steps are the same as in the flow chart illustrated in Fig. 18B, except that the fluidic patch 130 is not used. In the flow chart shown in Fig. 18C, the measurements made by the reusable biofluid computation measurement device 10 are not enhanced by the measurements made by the one-time use fluidic patch.

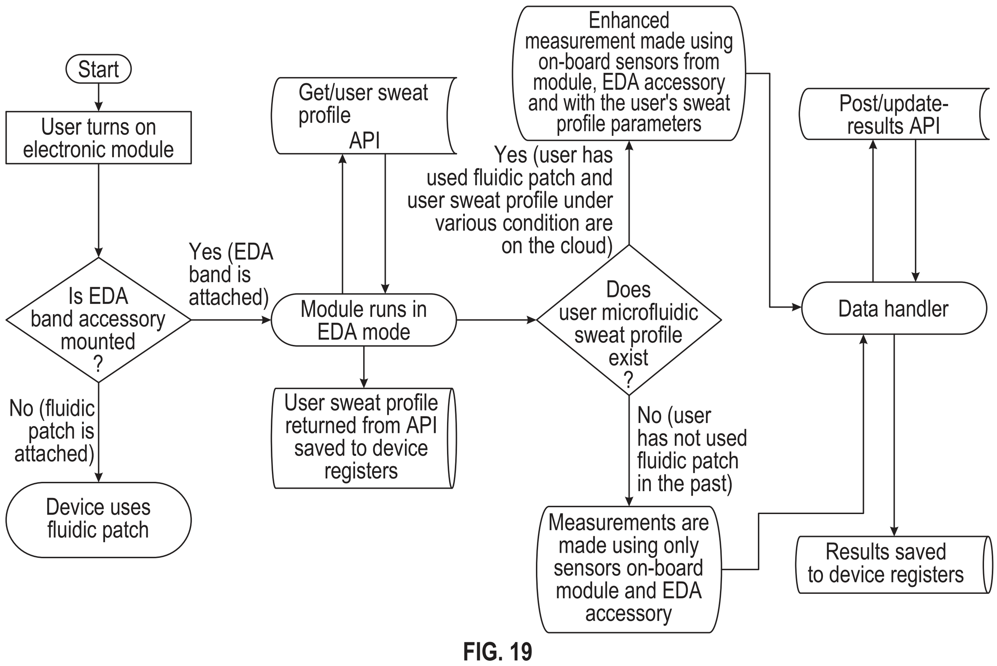

Fig. 19 is a data flow chart of the electronic module 12 initialization, identifying which accessory is attached, determining if a user sweat profile exists, and loading the user profile to perform an enhanced measurement.

A data flow chart illustrated in Fig. 19 shows how the electronic module 12 checks for an existing, historical, sweat profile for a user, and incorporates that profile to enhance the measurement. If the electronic module 12 does not have a historical sweat profile for the user, the electronic module 12 will rely solely on the built-in sensors to make a measurement.

Specifically, the electronic module 12 first determines that the EDA band assembly 14 is connected and then switches to EDA mode. The electronic module 12 then looks for an existing historical sweat profile logged from when the user used the fluidic patch 130. The sweat profile may be stored locally on the electronic module 12 or remotely on a network. If a profile exists, the electronic module 12 then accesses and uses the most recent profile. Data included in the user's sweat profile are parameters computed from past sessions that were measured explicitly using the fluidic patch 130, which measures fluid volume fill and electrolyte concentration. The parameters from the profile are fed into a sweat computation model used by the electronic module 12 when in EDA mode, which primarily measures skin conductance, simple volume (for example, the volume in a fixed volume reservoir), and electrolyte changes, to enhance the sweat loss computation. The results of the measurements are then saved.

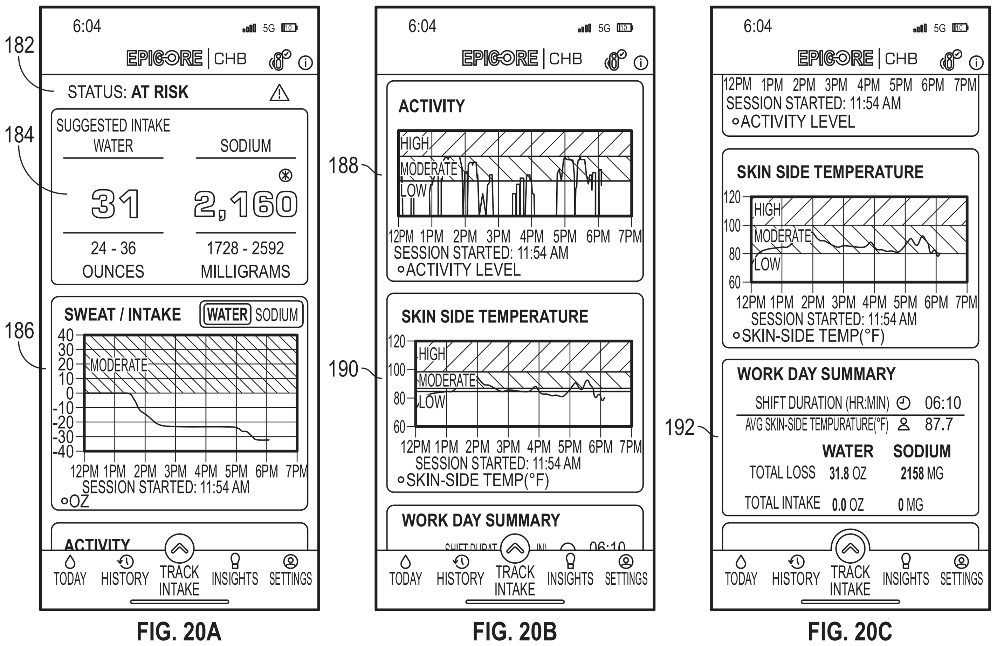

Figs. 20A, 20B, and 20C are plan views of first, second, and third portions, respectively, of a GUI App screen. Fig. 20A shows the wearer's current hydration status at 182, the suggested water and sodium intake at 184, and the water and sodium status over the course of the session at 186. Fig. 20B shows the wearer's activity level at 188, and the skin-side temperature over the course of the session at 190. Fig. 20C shows the session summary metrics at 192.

The mobile app's GUI takes cues from whichever accessory is connected to the electronic module 12. The electronic module 12 includes a microcontroller 127 and circuitry configured to recognize whether the fluidic patch 130 or the EDA band assembly 14 is connected. The mobile app's GUI can display respective, context-specific information in the mobile app. In addition, the mobile app can suggest a course of action using an accessory, i.e., either the fluidic patch 130 or the biofluid computation measurement device 10, that is currently not connected. If the electronic module 12 is connected to the EDA band assembly 14, the GUI shows the relevant contextual information from the EDA band assembly 14, but the mobile app can also offer tips and suggestions to switch to the other accessory, i.e., the fluidic patch 130.

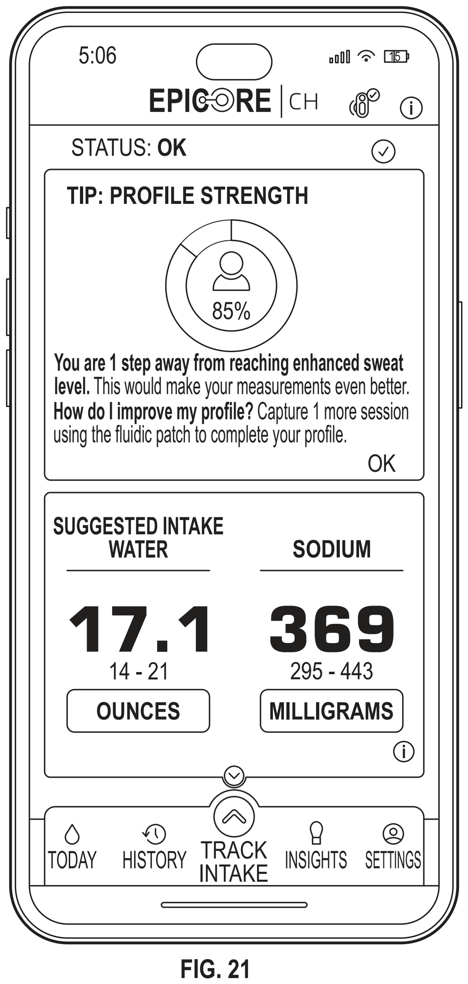

Advantageously, measurements made by the electronic module 12 and the EDA band assembly 14 may be enhanced with the use of the user's historical sweat profile obtained from prior measurements using the fluidic patch 130. The mobile app can promote building a sweat profile by nudging or guiding the user to first use the fluidic patch 130 for a few sessions to create a characteristic sweat profile of the user, as shown in Fig. 21. As shown in Fig. 21, a note in the form of a tip is provided on the mobile app's main screen telling the user how complete the profile is. In the embodiment of the mobile app GUI shown in Fig. 21, the note explains that completing one additional session with the fluidic patch 130 would complete the data needed to formulate a user's characteristic sweat profile. The user can then switch to using the EDA band assembly 14 in the next session. The characteristic profile may then be incorporated in the measurement algorithm when using the EDA band assembly 14, thus taking advantage of the sweat profile from prior microfluidic measurements, resulting in a more accurate measurement than when relying solely on skin conductance and limited-size reservoir volumes for changing sweat rates over long durations of time.

Conventional consumer wearable electronic devices often struggle because the data that they display is not actionable. Advantageously, the data collected from the electronic module 12, when used with biofluid computation measurement device 10 or with the fluidic patch 130 is made very actionable. For example, sweat loss data is used to generate a suggested replenishment of water and sodium as listed under the Suggested Intake heading in the mobile app as shown at 184 in Fig. 20A. Additional actionable data may also be displayed.

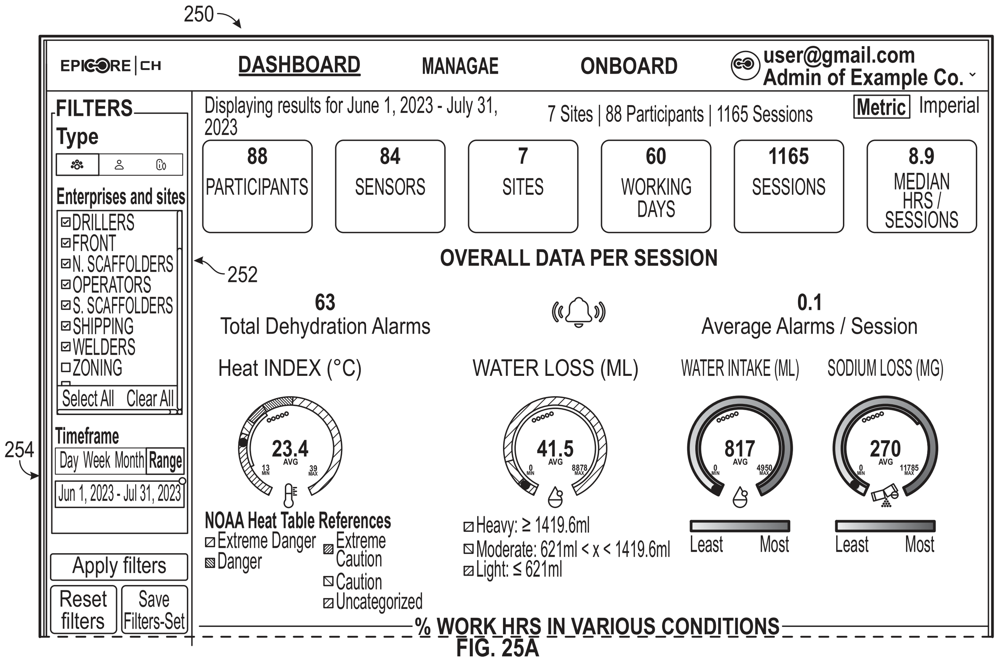

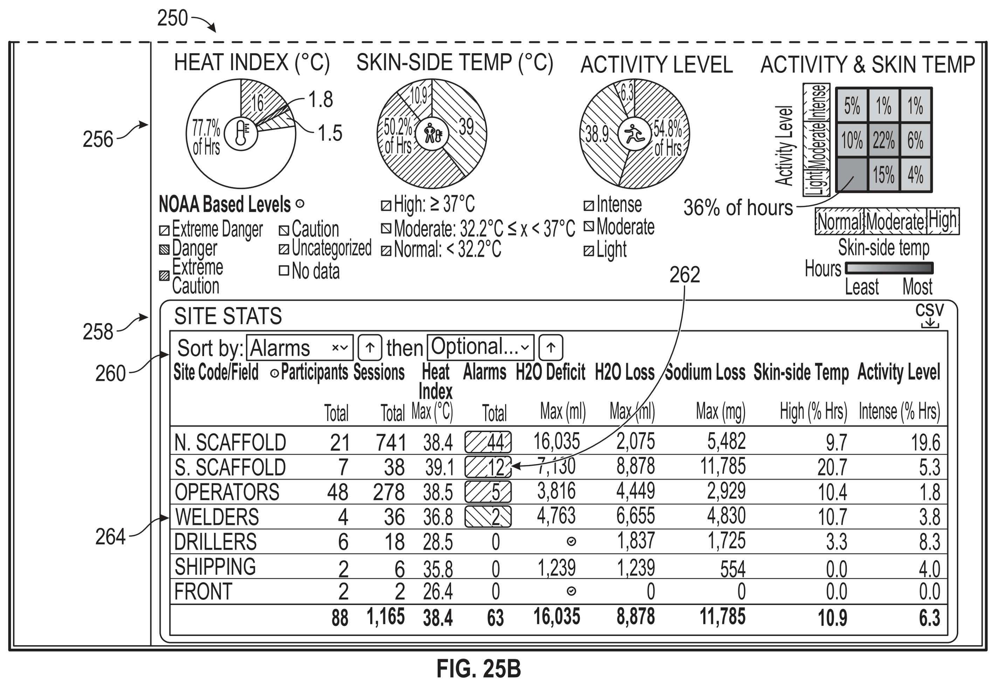

Figs. 25A and 25B are upper and lower halves, respectively, of a user screen 250 from cloud-based web portal. The cloud-based web portal user screen illustrated in Figs. 25A and 25B represents one embodiment of a visual presentation and organization of actionable data available when using the biofluid computation measurement device 10. The user screen 250 visually presents actionable data in the form of a site statistics table wherein sites are listed by risk. Additionally, the user screen 250 provides overview information as to the hydration practices and conditions of the workers listed therein.

The illustrated user screen 250 displays data organized such that occupational hygienists, health and safety personnel, or managers can take action to address shortcomings in hydration practices, or reduce risk of heat exposure. A user may: filter data by groups of workers (see 252), filter data by timeframe (see 254), view the percentage of time a group or an individual performed tasks in various working conditions, such as in an environment with a high heat index, where high skin temperatures are present, where high activity levels occur, and where both high skin temperatures and high activity levels occur (see 256). Additionally, the user can view a list of worker group statistics in a table format, and view the worker groups' exposure risk levels and their sweat loss and alarms (see 258). The user may also sort the data by any of a plurality of parameters (see 260), such as a 2% body mass dehydration alarm count (see 262), and create and view customized names of worker groups (see 264).

In a work environment, the biofluid computation measurement device 10 may be used to organize individual workers into groups, such as but not limited to, geographic, or type of task. Such groups are conducive for the health staff member to visit and provide training/feedback. The information for each worker group may be organized in any desired manner, such as in the table as shown at 258 in Fig. 25B, wherein groupings may be organized by risk as shown at 262. The biofluid computation measurement device 10 may include an alarm to alert a user when the user loses sweat that has not been replenished resulting in a loss of 2% of the user's body mass. This amount has been shown to indicate the onset of cognitive decline. Preventing dehydration is only one aspect of biofluid computation measurement device 10. Keeping users mentally sharp and high-performing is another important aspect. Additionally, the biofluid computation measurement device 10 may be configured to measure at least one of humidity, heat flux, and pulse rate via photoplethysmography.

The organization and presentation of data as group data rather than individual data has additional benefits. Workers may be sensitive to the type of personal data that is shown to employers, even health staff. There may be labor laws that protect employment rights of workers. Organizing collected data as group data de-identifies individual worker data and only presents group statistics. Health and safety professionals can monitor the work groups where each worker's data is anonymous, but the group's data can be easily viewed. The group's hydration practices may be scrutinized. Those groups who rehydrate well may be rewarded and those groups who do not rehydrate well and have risky practices may be identified for additional safety training. The list makes it easy for health and safety staff to address teams and take effective action, such as through better training or improvements in processes, without singling out individuals.

The physiological data of the group includes, but is not limited to, sweat deficit, which is defined as sweat loss minus fluid intake, sweat deficit per session, which is defined as sweat loss minus fluid intake divided by number of sessions, a percentage of activity at high levels, a percentage of skin temperature at high levels, heat exposure, skin-temperature, and alarms, organized in a roster format where subjects are not shown as individuals but are organized into risk-ranked groups such that health and safety professionals can take action to address high risk behavior. For example, the health and safety professionals may provide safety training to groups who show high risk behavior. In another example, the health and safety professionals may change shift schedules to reduce exposure to heat.

When viewed comprehensively as a system, such as described in Fig. 18B, the electronic module 12 is able to obtain data from either the fluidic patch 130 that can measure sweat volume changes over long time durations with its extensive microfluidic channel, or from the convenient and re-usable EDA band assembly 14. As described above, the fluidic patch 130 is able to measure direct sweat volume for longer periods of time because the microfluidic channel 138 therein is relatively long. In contrast, the embodiments of the electronic substrate described herein for use in the EDA band assembly 14 have relatively shallow reservoirs that fill with sweat over a shorter period of time. The ability to develop an individual's characteristic sweat profile with the features of one accessory, for example, the fluidic patch 130, and apply that profile in the form of characteristic coefficients to improve the measurement of another accessory, for example, the more convenient-to-wear re-usable EDA band assembly 14, enables the biofluid computation measurement device 10 to offer the best of both types of measurement methods while maintaining comfort.

It will be understood that data from the re-usable electronic module 12 may be transmitted across a computer network, and may be viewed on a web portal.

The embodiments of the sweat rate, volume, and electrolyte measurement system disclosed herein include a notification system such that when a plurality of the one-time use fluidic patch 130 and/or the wearable biofluid computation measurement device 10 are being monitored by a central administrator, the central administrator may send a notification to either a wearer or a group of wearers to provide instructions to perform an action. Such actions for which instructions are provided may include a notice to return the one-time use fluidic patches 130 and/or the wearable biofluid computation measurement devices 10 for maintenance, a notice to an administrator to collect the one-time use fluidic patches 130 and/or the wearable biofluid computation measurement devices 10 and arrange for maintenance with the manufacturer or a service provider, and a notice to the service provider to follow-up with the administrator for maintenance.

The embodiments of the sweat rate, volume, and electrolyte measurement system disclosed herein further include a notification system that provides notifications through algorithms to inform wearers to act with caution in anticipation of a known or anticipated event. Such known or anticipated events may include inclement weather, a pending heat wave, the risk of injury based on an assessment of future activity, such as planned, high-intensity physical activity in hot weather, and known historic behavior of the wearer, such as propensity to dehydrate due to not rehydrating appropriately.

The following numbered statements form part of the present disclosure:

Statement 1. An electrode substrate, configured to be pressed onto the skin of a wearer, comprising:

a body having a first, skin-facing side and a second side that faces away from the skin;
a plurality of raised platforms extending outwardly from the skin-facing side;
recessed areas formed between each of the raised platforms and defining air gaps for the flow of air between each raised platform; and
a skin-facing electrode formed on each of at least two of the plurality of raised platforms;
wherein the skin-facing electrodes are planar and flush with an outer surface of the raised platforms;
wherein the electrode substrate and the skin-facing electrodes formed thereon are configured to measure change in impedance of the skin onto which it is pressed over time, including the time before, during, and after sweat perfusion; and
wherein edges of the raised platforms are rounded for comfort when worn and pressed into the skin of the wearer.

Statement 2. The electrode substrate according to Statement 1, further including a PCB mounted within the body.

Statement 3. The electrode substrate according to Statement 1 or 2, wherein the body is formed from one of ceramic and/or an injection molded polymer; and wherein the skin-facing electrodes are formed from one of electroplating and vapor deposition.

Statement 4. The electrode substrate according to any of the preceding Statements, wherein the skin-facing electrodes are configured to excite with frequencies within the range of about 100 Hz to about 100 kHz.

Statement 5. The electrode substrate according to any of the preceding Statements, wherein the skin-facing side has a fixed volume reservoir formed therein.

Statement 6. The electrode substrate according to Statement 5, wherein the fixed volume reservoir is formed in one of the plurality of raised platforms.

Statement 7. The electrode substrate according to Statement 5 or 6, wherein the fixed volume reservoir has an outlet hole formed therein; and wherein the outlet hole has a size sufficiently small that surface tension of sweat in the reservoir prevents the sweat from leaving the reservoir until the reservoir has completely filled.

Statement 8. The electrode substrate according to any of Statements 5 to 7, wherein an outer, peripheral edge of the fixed volume reservoir defines an inlet port and is configured such that when the skin-facing side of the electrode substrate is pressed into the skin of the wearer, a watertight seal is formed.

Statement 9. The electrode substrate according to Statement 8, wherein the outer, peripheral edge of the fixed volume reservoir includes an outwardly extending lip;
wherein the lip is rounded for comfort when the electrode substrate is worn and pressed into the skin of the wearer.

Statement 10. The electrode substrate according to any of Statements 5 to 9, wherein a pair of electrodes are formed the fixed volume reservoir;

wherein the pair of electrodes do not make contact with skin and only make contact with perfused sweat when the fixed volume reservoir fills with sweat;
wherein the pair of electrodes is configured to measure conductance in a known volume of sweat inside the reservoir; and
wherein the pair of electrodes are formed in a lower surface of the fixed volume reservoir.

Statement 11. The electrode substrate according to any of the preceding Statements, wherein the skin-facing electrode formed on each of at least two of the plurality of raised platforms define a first pair of electrodes, the electrode substrate further including a second pair of electrodes; and
wherein the second pair of electrodes measures impedance of sweat that forms on the surface of the top layers of the wearer's skin.

Statement 12. The electrode substrate according to Statement 11, wherein the second pair of electrodes have a smaller pitch and a smaller surface area than the first pair of electrodes.

Statement 13. The electrode substrate according to any of the preceding Statements, further including an electrical resistance device that operates independently of temperature and humidity.

Statement 14. The electrode substrate according to any of the preceding Statements, further including an electrical resistance device that measures one of temperature and humidity.

Statement 15. A sweat rate, volume, and electrolyte measurement system comprising:
a wearable biofluid computation measurement device including:

a re-usable electro-dermal activity (EDA) band assembly having an electrode substrate mounted within a substrate housing portion of a clip, and a compression strap attached to the clip; and
re-usable electronic module attached to the EDA band assembly; and
a smart device;
wherein the electrode substrate has at least one pair of a skin-facing electrodes formed thereon; and
wherein the electronic module includes one of a haptic alert and an alarm that activates when the sensor readings from the electrode substrate exceed a pre-determined threshold measurements including, but not limited to, sweat volume, sweat deficit, defined as sweat volume lost minus fluid intake, and core-temperature; and
wherein the measurement taken by the electrode substrate are wirelessly transmitted to the smart device.

Statement 16. The sweat rate, volume, and electrolyte measurement system according to Statement 15, wherein the electrode substrate is configured to be pressed onto the skin of a wearer and includes:

a body having a first, skin-facing side and a second side that faces away from the skin;
a plurality of raised platforms extending outwardly from the skin-facing side;
recessed areas formed between each of the raised platforms and defining air gaps for the flow of air between each raised platform; and
a skin-facing electrode formed on each of at least two of the plurality of raised platforms;
wherein the skin-facing electrodes are planar and flush with an outer surface of the raised platforms; and
wherein the electrode substrate and the skin-facing electrodes formed thereon is configured to measure change in impedance of the skin onto which it is pressed over time, including the time before, during, and after sweat perfusion.

Statement 17. The sweat rate, volume, and electrolyte measurement system according to Statement 15 or 16 further including a one-time use fluidic patch having:

a first, skin-facing side and a second side that faces away from the skin; striated patterned adhesive on the surface of the skin-facing side;
a microfluidic channel within the fluidic patch, the microfluidic channel having an inlet port and an outlet port;
wherein perfused sweat enters the microfluidic channel through the inlet port and exits through the outlet port;
a plurality of electrodes within the microfluidic channel, wherein the plurality of electrodes are in contact with the captured perfused sweat within the microfluidic channel, and wherein the electrodes are configured to measure fluid volume and fluid conductivity.

Statement 18. The sweat rate, volume, and electrolyte measurement system according to Statement 17, further including the re-usable electronic module attached to the one-time use fluidic patch.

Statement 19. The sweat rate, volume, and electrolyte measurement system according to Statement 18, wherein the one-time use fluidic patch and the wearable biofluid computation measurement device may be used separately or sequentially; and
wherein the re-usable electronic module can determine to which of the one-time use fluidic patch and the wearable biofluid computation measurement device the re-usable electronic module is connected.

Statement 20. The sweat rate, volume, and electrolyte measurement system according to Statement 18 or 19, wherein the sweat rate, volume, and electrolyte measurement system may have a first configuration such that the one-time use fluidic patch is applied to a wearer and used to measure the wearer's activity, temperature, sweat volume, and electrolyte captured levels to generate a characteristic sweat profile consisting of coefficients from the correlation of sweat loss for given activity, temperature, and time duration;

wherein the one-time use fluidic patch is disconnected from the re-usable electronic module, and re-usable electronic module is attached to the wearable biofluid computation measurement device;
wherein the wearable biofluid computation measurement device is applied to a wearer, such that the skin-facing electrodes measure sweat onset, sweat rate, and electrolyte conductivity; and
wherein measurement computation from the wearable biofluid computation measurement device may be augmented with the characteristic sweat profile developed from the one-time use fluidic patch.

Statement 21. The sweat rate, volume, and electrolyte measurement system according to any of Statements 15 to 20, wherein the smart device includes a mobile app having a graphical user interface (GUI) that displays contextual information related to the one of the one-time use fluidic patch and the wearable biofluid computation measurement device connected to the module.

Statement 22. The sweat rate, volume, and electrolyte measurement system according to any of Statements 15 to 21, wherein the smart device includes a mobile app having a graphical user interface (GUI) that displays a suggested a course of action using either one of the one of the one-time use fluidic patch and the wearable biofluid computation measurement device connected to the module, whether connected to the electronic module or not.

Statement 23. The sweat rate, volume, and electrolyte measurement system according to any of Statements 15 to 22, wherein the sweat rate, volume, and electrolyte measurement system is configured to measure at least one of humidity and heat flux, and generate a photoplethysmogram.

Statement 24. The sweat rate, volume, and electrolyte measurement system according to any of Statements 15 to 23, wherein the sweat rate, volume, and electrolyte measurement system is configured to be used for the organization and presentation of group data for a group of subjects.

Statement 25. The sweat rate, volume, and electrolyte measurement system according to any of Statements 15 to 24, wherein data from the re-usable electronic module may be transmitted across a computer network; and
wherein data from a plurality of the re-usable electronic modules may be viewed on a web portal.

Statement 26. The sweat rate, volume, and electrolyte measurement system according to any of Statements 15 to 25, further including a notification system such that when a plurality of the one-time use fluidic patch and/or the wearable biofluid computation measurement device are being monitored by a central administrator, the central administrator may send a notification to one of a wearer or a group of wearers to provide instructions to perform an action.

Statement 27. The sweat rate, volume, and electrolyte measurement system according to Statement 26, wherein the action for which instructions are provided include one of a notice to return the one-time use fluidic patches and/or the wearable biofluid computation measurement devices for maintenance, notice to an administrator to collect the one-time use fluidic patches and/or the wearable biofluid computation measurement devices and arrange for maintenance with the manufacturer or a service provider, and notice to the service provider to follow-up with the administrator for maintenance.

Statement 28. The sweat rate, volume, and electrolyte measurement system according to any of Statements 15 to 27, further including a notification system such that the sweat rate, volume, and electrolyte measurement system provides notifications through algorithms to inform wearers to act with caution in anticipation of a known or anticipated event;
wherein the known or anticipated event includes one of inclement weather, a pending heat wave, the risk of injury based on an assessment of future activity, such as planned, high-intensity physical activity in hot weather, and known historic behavior of the wearer, such as propensity to dehydrate due to not rehydrating appropriately.

Statement 29. The sweat rate, volume, and electrolyte measurement system according to any of Statements 15 to 28, further including a notification system such that when a plurality of the wearable biofluid computation measurement devices are being monitored by a central administrator, the central administrator may send a notification to one of a wearer or a group of wearers to provide instructions to perform an action;

wherein the notification system is further configured to present groups of subjects such that physiological data, a percentage of activity at high levels, a percentage of skin temperature at high levels, heat exposure, skin-temperature, and alarms, are presented in a roster format;
wherein subjects are organized into risk-ranked groups such that the instructions to perform an action are presented to health and safety members able to take action to address identified high risk behavior; and
wherein the physiological data presented includes one of sweat deficit and sweat deficit per session.

Statement 30. The sweat rate, volume, and electrolyte measurement system according to any of Statements 15 to 29, further including a smart device graphical user interface that recommends that a user complete a course of action described;

wherein instructions to the user include describing a sensor attachment to use to complete the recommended course of action; and
wherein the course of action includes identifying specific steps to accomplish, and promoted to the user as an invitation to complete a characteristic sweat profile of the user.

Statement 31. A sweat rate, volume, and electrolyte measurement system comprising the electrode substrate according to any of Statements 1 to 14.

The principle and mode of operation of this invention have been explained and illustrated in its preferred embodiment. However, it must be understood that this invention may be practiced otherwise than as specifically explained and illustrated without departing from its spirit or scope.

**Claims**

1. An electrode substrate, configured to be pressed onto the skin of a wearer, comprising:

   a body having a first, skin-facing side and a second side that faces away from the skin;
   a plurality of raised platforms extending outwardly from the skin-facing side;
   recessed areas formed between each of the raised platforms and defining air gaps for the flow of air between each raised platform; and
   a skin-facing electrode formed on each of at least two of the plurality of raised platforms;
   wherein the skin-facing electrodes are planar and flush with an outer surface of the raised platforms;
   wherein the electrode substrate and the skin-facing electrodes formed thereon are configured to measure change in impedance of the skin onto which it is pressed over time, including the time before, during, and after sweat perfusion; and
   wherein edges of the raised platforms are rounded for comfort when worn and pressed into the skin of the wearer.

2. The electrode substrate according to Claim 1, further including a PCB mounted within the body.

3. The electrode substrate according to Claim 1 or 2, wherein the body is formed from one of ceramic and/or an injection molded polymer; and wherein the skin-facing electrodes are formed from one of electroplating and vapor deposition.

4. The electrode substrate according to any of the preceding Claims, wherein the skin-facing electrodes are configured to excite with frequencies within the range of about 100 Hz to about 100 kHz.

5. The electrode substrate according to any of the preceding Claims, wherein the skin-facing side has a fixed volume reservoir formed therein.

6. The electrode substrate according to Claim 5, wherein the fixed volume reservoir is formed in one of the plurality of raised platforms.

7. The electrode substrate according to Claim 5 or 6, wherein the fixed volume reservoir has an outlet hole formed therein; and
   wherein the outlet hole has a size sufficiently small that surface tension of sweat in the reservoir prevents the sweat from leaving the reservoir until the reservoir has completely filled.

8. The electrode substrate according to any of Claims 5 to 7, wherein an outer, peripheral edge of the fixed volume reservoir defines an inlet port and is configured such that when the skin-facing side of the electrode substrate is pressed into the skin of the wearer, a watertight seal is formed.

9. The electrode substrate according to Claim 8, wherein the outer, peripheral edge of the fixed volume reservoir includes an outwardly extending lip;
   wherein the lip is rounded for comfort when the electrode substrate is worn and pressed into the skin of the wearer.

10. The electrode substrate according to any of Claims 5 to 9, wherein a

    pair of electrodes are formed the fixed volume reservoir;
    wherein the pair of electrodes do not make contact with skin and only make contact with perfused sweat when the fixed volume reservoir fills with sweat;
    wherein the pair of electrodes is configured to measure conductance in a known volume of sweat inside the reservoir; and
    wherein the pair of electrodes are formed in a lower surface of the fixed volume reservoir.

11. The electrode substrate according to any of the preceding Claims, wherein the skin-facing electrode formed on each of at least two of the plurality of raised platforms define a first pair of electrodes, the electrode substrate further including a second pair of electrodes; and
    wherein the second pair of electrodes measures impedance of sweat that forms on the surface of the top layers of the wearer's skin.

12. The electrode substrate according to Claim 11, wherein the second pair of electrodes have a smaller pitch and a smaller surface area than the first pair of electrodes.

13. The electrode substrate according to any of the preceding Claims, further including an electrical resistance device that operates independently of temperature and humidity.

14. The electrode substrate according to any of the preceding Claims, further including an electrical resistance device that measures one of temperature and humidity.

15. A sweat rate, volume, and electrolyte measurement system comprising the electrode substrate according to any of the preceding Claims.

**FIG. 1**

**FIG. 2**

FIG. 3A

FIG. 3B

**FIG. 3C**

**FIG. 3D**

12

114

12B

42

FIG. 4B

114

12A

12

FIG. 4A

130

136
136
138
133
132
134

**FIG. 5B**

135
137

**FIG. 5A**

130

**FIG. 6**

**FIG. 7**

**FIG. 8**

**FIG. 9**

**FIG. 10**

FIG. 11

**FIG. 12**

FIG. 13

Legend:
- Ch1 (2mm Gap)
- Ch2 (16mm Gap)
- Ch4 (40mm Gap)

Y-axis: Voltage (mV)
X-axis: Time (seconds)

**FIG. 14**

**FIG. 15**

Comparison of Sodium Cavity Plateau Voltage vs Sodium Concentration Truth Measurement
Across Multiple Subjects

FIG. 16A

EP 4 732 766 A1

FIG. 16B
Subject A (42mM)

FIG. 16C
Subject B (72 mM)

FIG. 16D
Subject C (81 mM)

FIG. 16E
Subject D (106 mM)

**Skin-facing Electrode Conductance Signal vs. Time**
**(Constant Saline Perfusion)**

Legend:
- 16.1mM
- 33.4mM
- 44.7mM
- 58.1mM
- 76.8mM
- 88.3mM

Y-axis: Voltage (mV)

X-axis: Elapsed Time (s)

**FIG. 17**

EP 4 732 766 A1

EP 4 732 766 A1

**Disposable Only Flow**

12    130    180

| Before First Use | Work Shift | | After Shift |
|---|---|---|---|
| App On-Boarding and IFU | Connect Module to Fluidic, Apply Device, Pair to App | Acknowledge Alerts, Log Fluid Intake | Remove Device, Dispose of Fluidic, Power off Module |

Repeats Daily

**FIG. 18A**

**"Training" Flow**

12    130    15    180

| Before First Use | Work Shift | | After Shift |
|---|---|---|---|
| App On-Boarding and IFU | Connect Module to Fluidic, Apply Device, Pair to App | Acknowledge Alerts, Log Fluid Intake | Remove Device, Dispose of Fluidic, Power off Module |

Repeats Until End of "Training"

| After Shift | Work Shift | |
|---|---|---|
| Remove Device, Power off, Store | Acknowledge Alerts, Log Fluid Intake | Connect Module to Re-usable Clip, Apply Device, Pair to App |

Repeats Daily

**FIG. 18B**

**Reusable Only Flow**

12   15   180

**Before First Use**

App On-Boarding and IFU

**First Use**

Connect Module to Re-usable Clip, Apply Device, Pair to App

Acknowledge Alerts, Log Fluid Intake

**After Shift**

Remove Device, Power off, Store

Repeats Daily

**FIG. 18C**

FIG. 19

**FIG. 20A**

6:04    5G   EPICORE | CHB

STATUS: **AT RISK**

182

SUGGESTED INTAKE

184

WATER    SODIUM

31   2,160

24 - 36    1728 - 2592

OUNCES    MILLIGRAMS

186

SWEAT / INTAKE   [WATER] SODIUM

40
30
20
10
-10
-20
-30
-40
MODERATE

12PM 1PM 2PM 3PM 4PM 5PM 6PM 7PM
SESSION STARTED: 11:54 AM
∘OZ

ACTIVITY

TODAY   HISTORY   TRACK INTAKE   INSIGHTS   SETTINGS

**FIG. 20B**

6:04    5G   EPICORE | CHB

188

ACTIVITY

HIGH
MODERATE
LOW

12PM 1PM 2PM 3PM 4PM 5PM 6PM 7PM
SESSION STARTED: 11:54 AM
∘ACTIVITY LEVEL

190

SKIN SIDE TEMPERATURE

120
100
80
60
HIGH
MODERATE
LOW

12PM 1PM 2PM 3PM 4PM 5PM 6PM 7PM
SESSION STARTED: 11:54 AM
∘SKIN-SIDE TEMP(°F)

WORK DAY SUMMARY

SHIFT DURAT... 06:10

TODAY   HISTORY   TRACK INTAKE   INSIGHTS   SETTINGS

**FIG. 20C**

6:04    5G   EPICORE | CHB

12PM 1PM 2PM 3PM 4PM 5PM 6PM 7PM
SESSION STARTED: 11:54 AM
∘ACTIVITY LEVEL

SKIN SIDE TEMPERATURE

120
100
80
60
HIGH
MODERATE
LOW

12PM 1PM 2PM 3PM 4PM 5PM 6PM 7PM
SESSION STARTED: 11:54 AM
∘SKIN-SIDE TEMP(°F)

WORK DAY SUMMARY

SHIFT DURATION (HR:MIN) 06:10
AVG SKIN-SIDE TEMPURATURE(°F) 87.7

WATER    SODIUM

TOTAL LOSS   31.8 OZ    2158 MG

TOTAL INTAKE   0.0 OZ    0 MG

TODAY   HISTORY   TRACK INTAKE   INSIGHTS   SETTINGS

192

**FIG. 21**

FIG. 22

FIG. 23

FIG. 24D

FIG. 24C

FIG. 24B

FIG. 24A

**FIG. 25A**

250

EPICORE|CH    **DASHBOARD**    MANAGAE    ONBOARD    ⊙⊙ user@gmail.com / Admin of Example Co. ⌄

FILTERS

Type

Enterprises and sites
- ☑ DRILLERS
- ☑ FRONT
- ☑ N. SCAFFOLDERS
- ☑ OPERATORS
- ☑ S. SCAFFOLDERS
- ☑ SHIPPING
- ☑ WELDERS
- ☐ ZONING

Select All   Clear All

Timeframe

Day Week Month **Range**

Jun 1, 2023 - Jul 31, 2023

Apply filters

Reset filters   Save Filters-Set

252

254

Displaying results for June 1, 2023 - July 31, 2023     7 Sites | 88 Participants | 1165 Sessions     **Metric** Imperial

| 88 PARTICIPANTS | 84 SENSORS | 7 SITES | 60 WORKING DAYS | 1165 SESSIONS | 8.9 MEDIAN HRS / SESSIONS |
|---|---|---|---|---|---|

**OVERALL DATA PER SESSION**

63 — Total Dehydration Alarms     🔔     0.1 — Average Alarms / Session

Heat INDEX (°C)     WATER LOSS (ML)     WATER INTAKE (ML)     SODIUM LOSS (MG)

23.4 AVG   (13 MIN — 39 MAX)     41.5 AVG   (0 MIN — 8878 MAX)     817 AVG   (0 MIN — 4950 MAX)     270 AVG   (0 MIN — 11785 MAX)

NOAA Heat Table References
- ▨ Extreme Danger
- ▨ Danger
- ▨ Extreme Caution
- ☐ Caution
- ☑ Uncategorized

- ☑ Heavy: ≥ 1419.6ml
- ◪ Moderate: 621ml < x < 1419.6ml
- ☑ Light: ≤ 621ml

Least    Most          Least    Most

% WORK HRS IN VARIOUS CONDITIONS

FIG. 25A

EP 4 732 766 A1

EP 4 732 766 A1

250

## HEAT INDEX (°C)

77.7% of Hrs
16
1.8
1.5

**NOAA Based Levels** ⊙

▨ Extreme Danger  ▢ Caution
▧ Danger  ▨ Uncategorized
▨ Extreme Caution  ▢ No data

## SKIN-SIDE TEMP (°C)

50.2% of Hrs
10.9
39

▨ High: ≥ 37°C
▨ Moderate: 32.2°C ≤ x < 37°C
▨ Normal: < 32.2°C

## ACTIVITY LEVEL

38.9
6.3
54.8% of Hrs

▨ Intense
▨ Moderate
▨ Light

## ACTIVITY & SKIN TEMP

| Activity Level | | | |
|---|---|---|---|
| Intense | 5% | 1% | 1% |
| Moderate | 10% | 22% | 6% |
| Light | | 15% | 4% |

36% of hours

| Normal | Moderate | High |

Skin-side temp

Hours

Least — Most

256
258
260
264
262

### SITE STATS

CSV

Sort by: [Alarms ×⌄] [↑] then [Optional...⌄] [↑]

| Site Code/Field ⊙ | Participants | Sessions | Heat Index | Alarms | H2O Deficit | H2O Loss | Sodium Loss | Skin-side Temp | Activity Level |
|---|---|---|---|---|---|---|---|---|---|
| | Total | Total | Max (°C) | Total | Max (ml) | Max (ml) | Max (mg) | High (% Hrs) | Intense (% Hrs) |
| N. SCAFFOLD | 21 | 741 | 38.4 | 44 | 16,035 | 2,075 | 5,482 | 9.7 | 19.6 |
| S. SCAFFOLD | 7 | 38 | 39.1 | 12 | 7,130 | 8,878 | 11,785 | 20.7 | 5.3 |
| OPERATORS | 48 | 278 | 38.5 | 5 | 3,816 | 4,449 | 2,929 | 10.4 | 1.8 |
| WELDERS | 4 | 36 | 36.8 | 2 | 4,763 | 6,655 | 4,830 | 10.7 | 3.8 |
| DRILLERS | 6 | 18 | 28.5 | 0 | ⊙ | 1,837 | 1,725 | 3.3 | 8.3 |
| SHIPPING | 2 | 6 | 35.8 | 0 | 1,239 | 1,239 | 554 | 0.0 | 4.0 |
| FRONT | 2 | 2 | 26.4 | 0 | ⊙ | 0 | 0 | 0.0 | 0.0 |
| | 88 | 1,165 | 38.4 | 63 | 16,035 | 8,878 | 11,785 | 10.9 | 6.3 |

**FIG. 25B**

## EUROPEAN SEARCH REPORT

**Application Number**

EP 25 21 1687

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/020966 A1 (BEGTRUP GAVI [US] ET AL) 25 January 2018 (2018-01-25) | 1,2,5-15 | INV.<br>A61B5/053 |
| Y | * paragraphs [0039], [0047], [0060], [0063], [0075], [0076], [0109]; claim 9; figures 14, 14a * | 3,4 | A61B5/145<br>A61B5/01 |
| Y | WO 2022/164492 A1 (ANEXA LABS LLC [US]) 4 August 2022 (2022-08-04) | 3 | |
| A | * paragraphs [0063], [0109]; figure 9 * | 12 | |
| A | US 2018/192911 A1 (JUNG SUK WON [KR] ET AL) 12 July 2018 (2018-07-12) * paragraph [0060] * | 3 | |
| A | KR 101 244 816 B1 (UNIV KEIMYUNG IACF [KR]) 18 March 2013 (2013-03-18) * paragraph [0038] * | 3 | |
| Y | KR 200 483 102 Y1 (YUJEONG SYSTEM) 6 April 2017 (2017-04-06) * claim 1 * | 4 | |
| A | Anonymous: "11.3 Resistance and Resistivity - Douglas College Physics 1207", , 22 August 2016 (2016-08-22), XP093351125, Retrieved from the Internet: URL:https://pressbooks.bccampus.ca/introductorygeneralphysics2phys1207opticsfirst/chapter/20-3-resistance-and-resistivity/ * the whole document * | 13 | **TECHNICAL FIELDS SEARCHED (IPC)**<br>A61B |

The present search report has been drawn up for all claims

| Place of search | Date of completion of the search | Examiner |
|---|---|---|
| Berlin | 19 January 2026 | Clevorn, Jens |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 21 1687

This annex lists the patent family members relating to the patent documents cited in the above-mentioned European search report.
The members are as contained in the European Patent Office EDP file on
The European Patent Office is in no way liable for these particulars which are merely given for the purpose of information.

19-01-2026

| Patent document cited in search report | | Publication date | Patent family member(s) | | Publication date |
|---|---|---|---|---|---|
| US 2018020966 | A1 | 25-01-2018 | CN | 110035690 A | 19-07-2019 |
| | | | EP | 3487390 A1 | 29-05-2019 |
| | | | US | 2018020966 A1 | 25-01-2018 |
| | | | WO | 2018017619 A1 | 25-01-2018 |
| WO 2022164492 | A1 | 04-08-2022 | US | 11207025 B1 | 28-12-2021 |
| | | | US | 2025017532 A1 | 16-01-2025 |
| | | | WO | 2022164492 A1 | 04-08-2022 |
| US 2018192911 | A1 | 12-07-2018 | JP | 2018110860 A | 19-07-2018 |
| | | | KR | 20180082227 A | 18-07-2018 |
| | | | US | 2018192911 A1 | 12-07-2018 |
| KR 101244816 | B1 | 18-03-2013 | KR | 20120055187 A | 31-05-2012 |
| | | | US | 2013231547 A1 | 05-09-2013 |
| | | | WO | 2012070756 A1 | 31-05-2012 |
| KR 200483102 | Y1 | 06-04-2017 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63712660
- US 11559225 B